(19) Europäisches Patentamt
European Patent Office
Office européen des brevets

(11) EP 3 909 613 A1

# (12) EUROPEAN PATENT APPLICATION

(43) Date of publication:
**17.11.2021 Bulletin 2021/46**

(51) Int Cl.:
***A61K 47/69** (2017.01)* ***A61P 35/00** (2006.01)*
***A61P 37/02** (2006.01)*

(21) Application number: **20196024.2**

(22) Date of filing: **14.09.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.05.2020 EP 20174156**

(71) Applicants:
- **Life Science Inkubator Betriebs GmbH & Co. KG 53175 Bonn (DE)**
- **DKFZ Deutsches Krebsforschungszentrum 69120 Heidelberg (DE)**

(72) Inventors:
- **KÜBELBECK, Armin 53175 Bonn (DE)**
- **RIEMER, Angelika 69120 Heidelberg (DE)**
- **KRUSE, Sebastian 69120 Heidelberg (DE)**
- **FEIDT, Eva 53175 Bonn (DE)**
- **GRABOWSKA, Agnieszka 53175 Bonn (DE)**
- **JUNGLAS, Ellen 53175 Bonn (DE)**

(74) Representative: **von Renesse, Dorothea et al
König-Szynka-Tilmann-von Renesse
Patentanwälte Partnerschaft mbB
Mönchenwerther Str. 11
40545 Düsseldorf (DE)**

## (54) NANOPARTICLES AS CARRIER-SYSTEM FOR ADJUVANTS/ANTIGENS

(57) The disclosure relates to immunological compositions (immunostimulants) based on nanoparticles as carrier for adjuvants, optionally in combination with antigens or epitopes, in particular for the use of the compositions for immunoprophylaxis or immunotherapy.



Printed by Jouve, 75001 PARIS (FR)

## Description

### FIELD OF THE INVENTION

The present invention relates to immunological compositions (immunostimulants) based on nanoparticles as carrier for adjuvants, optionally in combination with antigens or epitopes, in particular for the use of the compositions for immunoprophylaxis or immunotherapy.

### BACKGROUND OF THE INVENTION

Many molecules have been considered for use as an adjuvant, including mineral compounds (e.g. "Alum" = aluminum hydroxide gel), water-in-oil or oil-in-water emulsions (e.g. complete or incomplete Freund’s adjuvant), as well as natural and synthetic toxins derived from bacteria (e.g. cholera toxin, CT and lymphotoxin, LT). Based on their mechanism of action, adjuvants have been categorized into two broad groups; the particulate vaccine-delivery systems that target antigen presenting cells (APCs) and the immune stimulatory adjuvants that directly activate such cells through specific receptors e.g. toll-like receptors (TLRs) resulting in inflammatory responses that amplify the innate immune response. TLR ligands used as adjuvants can augment the immune response to vaccines through a variety of mechanisms. Therefore, the ultimate aim is to activate the innate immune system to respond more rapidly to immunization and for the adaptive immune response to become more specific.

Paramunity inducers are known to increase the non-specific immunity of the body, the para-immunity (paramunity). The complement system is activated, cytokines are released and various immune cells are activated. In contrast to specific immune stimulation with an antigen, paramunity is only a short-term immunity. It lasts for about 1 to 2 weeks, but starts after 6 to 8 hours.

Drugs with paramunizing properties that are preferred in human medicine for immunostimulation and as "adjuvant immunotherapy" are for example BCG (Bacillus Calmette-Guerin), Levamisole and Corynebacterium parvum (synonym Propionibacterium acnes).

A well-known paramunity inducer is PIND-ORF (trade name Zylexis®). PIND-ORF is produced from an attenuated parapoxvirus, strain D-1701. PIND-ORF is approved for use in pets and some livestock.

In 2015, the first commercial DNA immunostimulator product, ZelNate®, was introduced to the veterinary market. This product consists of bacterial plasmid DNA rich in non-methylated CpG motifs that are encased in a cationic liposome shell. ZelNate® is indicated for use as an aid in the treatment and prophylaxis of bovine respiratory disease due to *Mannheimia haemolytica* in 4 month-old cattle or older, when administered at the time of, or within 24 h after, a perceived stressful event.

Pathogen-associated molecular patterns (PAMPs) are increasingly recognized as a key component of many modern vaccines. Polyinosinic-polycytidic acid, poly(I:C) is a well-known TLR3 agonist and is one of the synthetic dsRNA molecules that activates innate and adaptive immune components. It mimics viral infections and elicits host immune responses by triggering specific pattern recognition receptors (PRRs).

Functionalized nanoparticles are well-known as drug-delivery systems, e.g. as carrier for antigens. The literature describes in particular carrier systems in which the antigens are either encapsulated or bound to the surface of the nanoparticles.

WO 2006/037979 A2 describes gold nanoparticles (GNPs) comprising adjuvants and antigens, such as tumor and pathogen antigens, and their use in a range of applications such as for the treatment of cancer and infectious diseases. Also disclosed are immunogenic structures based on nanoparticles or antibodies with carbohydrate ligands, and their use for therapeutic and prophylactic purposes, and for the isolation and detection of antibodies directed against the carbohydrate structures.

WO 2013/034741 A1 and WO 2013/034726 A1 relate to nanoparticles having an epitopic peptide bound via a linker and which find use as vaccines, e.g. in the prophylactic or therapeutic treatment of a tumor in a mammalian subject.

WO 2011/154711 A1 describes glycated gold nanoparticles that act as carriers for delivery of peptides such as insulin.

WO 2010/006753 A2 discloses monodisperse nanoparticles of silicon dioxide with at least one antigen attached to their surface. The nanoparticles are used for the immunoprophylaxis or immunotherapy of cancer.

Although many compositions based on nanoparticles have been investigated and developed in particular in the context of treating cancer there is still a high demand on providing substances with improved characteristics, in particular in terms of their adjuvant effects. There is also still the need for new effective immunostimulators for the treatment of humans and animals.

## SUMMARY OF THE INVENTION

It is thus objective of the present invention to provide improved compositions comprising nanoparticles in particular for use in immunoprophylaxis or immunotherapy. It is furthermore an objective of the invention to provide compositions which have effective immunomodulatory activities and could be used as an immune booster or as therapeutic or prophylactic vaccine.

These objectives are solved by providing a composition comprising nanoparticles with silicon dioxide and functional groups on the surface, which are loaded with pharmaceutically acceptable compounds comprising polyinosinic:polycytidylic acid, (poly(I:C)) or any derivatives thereof. Alternatively, the nanoparticles are loaded with poly(I:C) and an antigen or an epitope. The pharmaceutically acceptable compounds (poly(I:C) and antigen/epitope) represent the payload of the nanoparticles. The nanoparticles have a particle size below 150 nm. The functional groups on the surface of the nanoparticles are suitable for carrying and/or stabilizing negative and positive charges of such compounds. The composition has a zeta potential of at least $\pm$ 15 mV.

Poly(I:C) is a synthetic dsRNA that can activate multiple elements of the host defense in a pattern that parallels that of a viral infection. Derivatives of poly(I:C) are for example polyI:poly$C_{12}$U and poly-ICLC.

It was found that compositions according to the invention are able to activate multiple elements of innate immunity, to activate APCs and antigen processing, to generate polyfunctional cytotoxic T lymphocyte response, to target that response to tumors or pathogens via chemokines, to generate memory T and B cells, antibodies and long-term immunity.

Moreover, with a composition according to the invention one fundamental problem of nanoparticles for drug delivery is overcome, which is the lack of stability of the colloidal suspension even under physiological conditions.

In aqueous systems, surface charges essentially ensure the stability of colloidal systems. These charges can be positive or negative. To substantially reduce or even avoid agglomeration of the particles it is important that there are enough functionalities of the same charge, which leads to electrostatic repulsion. If the charge of the colloidal carrier system is compensated by the adsorption of the oppositely charged molecules, agglomerates will be formed and the colloidal system collapses.

The agglomerates have significantly larger particle diameters, which jeopardizes an effective transport of these large particles within the body and leads to a less effective immunomodulatory efficacy.

The composition according to the invention with nanoparticles having a particle size of below 150 nm and a zeta potential of at least $\pm$ 15 mV ensures that the composition is sufficiently stable. In particular under physiological conditions, the nanoparticles dispersed therein can effectively be transported to their site of main activity within the body. The compositions according to the invention thus allow the improved transport of the nanoparticles into the lymphatic system, in particular from the administration site to the lymph nodes in which dendritic cells are located.

When administered by subcutaneous injection the particles according to the invention are too large to enter the blood circulation, hence their fast elimination as well as severe systemic adverse effects are essentially avoided. At the same time they are small enough to penetrate into lymphatic vessels and to enter naïve dendritic cells, located in lymph nodes, by phagocytosis. The same applies when the particles are administered intradermally, intraperitoneally or intramuscularly. It is thus preferred to administer the particles parenterally with the exception of intravenous or intraarterial administration.

The inventors have found that the particles in the composition according to the invention can carry a high number of pharmaceutically acceptable compounds on the surface. The total amount of the compounds can be up to 5 % by weight with regard to the surface of the nanoparticle in $nm^2$ (surface loading density). The suitability of the nanoparticles to carry huge amounts of compounds is particularly important when high doses of such compounds (e.g. antigens or drug substances) are to be delivered.

The compositions according to the invention comprise nanoparticles with silicon dioxide and functional groups on the surface. The functional groups can be directly or indirectly connected to the surface of the nanoparticle. In a preferred embodiment of the invention the functional groups are connected to the surface of the nanoparticle via a linker (hereinafter linker compound L). The linker compound L can be connected to the surface of the nanoparticles by any way, in particular by covalent or adsorptive bond, most preferred by covalent bond.

In a particularly preferred embodiment the linker compound L comprises at least one functional group selected from the group consisting of carboxyl (-COOH) or carboxylate ($-COO^-$) group and/or at least one functional group selected from the group consisting of guanidino-group ($-NHC(=NH)NH_2$ or $-NHC(=NH_2^+)NH_2$) or amino-group ($-NH_2$ or $-NH_3^+$). Preferably, it comprises both, a carboxyl (-COOH) or carboxylate ($-COO^-$) group and at least one group selected from the group consisting of guanidino-group ($-NHC(=NH)NH_2$ or $-NHC(=NH_2^+)NH_2$) or amino-group ($-NH_2$ or - $NH_3^+$). Most preferred is that the linker compound L comprises a carboxyl (-COOH) or carboxylate ($-COO^-$) group and at least one guanidino-group ($-NHC(=NH)NH_2$ or - $NHC(=NH_2^+)NH_2$).

Such a linker compound L allows the adsorptive binding of the anionic compound poly(I:C). Therewith the linker provides a technology for the simple coupling of poly(I:C) or its derivatives and alternatively in combination with an

antigen or epitope to the surface of the nanoparticle.

## DETAILED DESCRIPTION OF THE INVENTION

The zeta potential of a nanoparticle is a commonly used parameter known to the skilled person to characterize the surface charge property of nanoparticles. It reflects the electrical potential of particles. To avoid agglomeration of the particles it is important that there are enough functionalities of the same charge which repel each other. Agglomerates cause the colloidal system to collapse. The resulting agglomerates have significantly larger particle diameters than the individual particles and therefore the desired transport mechanism via the fenestrated endothelium of the lymphatic vessels is no longer guaranteed. It is accepted as a measure for the stability of colloidal systems.

Current characterization methodologies are based on ensemble measurements (e.g. phase analysis light scattering, Doppler velocimetry, streaming potentiometry) that measure the average electrophoretic mobility of particles in suspension (Sci. Rep. 12 Dec. 2017; 7(1): 17479, PMCID: PMC5727177). It is particularly preferred to measure the zeta potential of the composition according to the invention with Dynamic Light Scattering (DLS) (ZetaSizer Nano ZS, Malvern Instruments, UK).

The zeta potential of the composition according to the present invention has a value of at least ± 15 mV, preferred ± 30 mV, more preferred ± 60 mV and most preferred between ± 25 and ± 40 mV. Therewith the composition, which is desirably a colloidal suspension, is stabilized, which means that the collapse of the system is essentially avoided.

The compositions according to the invention contain nanoparticles with a particle size below 150 nm, preferably 100 nm or less. More preferred are nanoparticles with a particle size of 50 nm or less, most preferred with a particle size between 20 and 30 nm. The particle size defined herein should be interpreted in such a way that a random distribution over the entire range is not present, but instead a defined particle size within the range is selected, of which the standard deviation is a maximum of 15%, preferably a maximum of 10%, wherein the standard deviation always relates to the local maximum in case of a bi- or multimodal distribution.

An indicator for the particle size of nanoparticles is the Z-average diameter. The Z-average diameter measured in dynamic light scattering is a parameter also known as the cumulant mean. It is the primary and most reliable parameter produced by the technique. The Z-average diameter is typically used in a quality control setting according to ISO 22412:2017. Dynamic light scattering techniques will give an intensity weighted distribution, where the contribution of each particle in the distribution relates to the intensity of light scattered by the particle. It is preferred to measure the intensity weighted distributions with a ZetaSizer Nano ZS (Malvern Instruments, UK).

In a preferred embodiment the Z-average diameter of the nanoparticles according to the invention is in the range of ≥5 and <150 nm, preferably ≥15 and ≤60nm, more preferably ≥20 and ≤40nm and still more preferably between 20 and 30nm, measured according to ISO 22412:2017.

The particle size of the nanoparticles and the stability of the composition according to the invention are furthermore confirmed by means of filtration with a sterile filter with maximum 0.2 μm pore size. However, this is practical only for nanoparticles with a diameter less than 50 nm.

The stability of the composition of the invention can be demonstrated by its polydispersity index (PDI). The PDI in general is an indicator for the uniformity of a system; in the context of the invention for the uniformity and stability of the colloidal nanoparticle suspension. The PDI reflects the nanoparticle size distribution. Samples with a wider range of particle sizes have higher PDI, while samples consisting of evenly sized particles have lower PDI. The skilled person is aware of methods and instruments for the measurement of the PDI, in particular a ZetaSizer Nano ZS (Malvern Instruments, UK).

A PDI greater than 0.7 indicate that the sample has a broad size distribution. A PDI below 0.1 is considered to be monodisperse. The various size distribution algorithms work with data that falls between these two extremes. The calculations for these parameters are defined in the ISO standard document 13321:1996 E and ISO 22412:2008.

The compositions according to the invention show a PDI between 0 and 0.32, preferably between 0.1 and 0.3, more preferably between 0.1 and 0.2, most preferred less than 0.1. The compositions according to the invention have a PDI less than 0.1 and are preferably monodisperse.

In a preferred embodiment the Z-average diameter of the nanoparticles according to the invention is in the range of ≥20 and ≤40nm and a PDI between 0.1 and 0.30 and a zeta potential between ± 20 and ± 40 mV

In a more preferred embodiment the Z-average diameter of the nanoparticles according to the invention is in the range of 20 and 30nm and a PDI between 0.1 and 0.2 and a zeta potential between ± 25 and ± 40 mV.

In a most preferred embodiment the Z-average diameter of the nanoparticles according to the invention is in the range of 20 and 30nm and a PDI less than 0.1 and a zeta potential between ± 25 and ± 40 mV.

It is also possible to measure the particle size of the loaded and unloaded particles by TEM or SEM.

As used herein, the term "transmission electron microscopy (TEM)" refers to a microscopy technique whereby a beam of electrons is transmitted through an ultra thin specimen, interacting with the specimen as it passes through it. An image is formed from the electrons transmitted through the specimen, magnified and focused by an objective lens

and appears on an imaging screen, a fluorescent screen in most TEMs, plus a monitor, or on a layer of photographic film, or to be detected by a sensor such as a CCD camera.

As used herein, the term "scanning electron microscope (SEM)" refers to a type of electron microscope that produces images of a sample by scanning the surface with a focused beam of electrons. The electrons interact with atoms in the sample, producing various signals that contain information about the surface topography and composition of the sample. The electron beam is scanned in a raster scan pattern, and the position of the beam is combined with the intensity of the detected signal to produce an image.

In **Fig. 1a** to **1d** show TEM images of the following $SiO_2$ particles:

**Fig. 1a** shows a TEM image of $SiO_2$ nanoparticles with a particle size of 68 nm
**Fig. 1b** shows a TEM image of $SiO_2$ nanoparticles with a particle size of 40 nm
**Fig. 1c** shows a TEM image of $SiO_2$ nanoparticles with a particle size of 25 nm
**Fig. 1d** shows a TEM image of $SiO_2$ nanoparticles with a particle size of 15 nm
**Fig. 2** shows a SEM of $SiO_2$ nanoparticles with a particle size of 150 nm.

In connection with the present invention, a "nanoparticle" is taken to mean a particulate binding matrix which has functionalities on its surface which function as recognition points for pharmaceutically acceptable compounds, e.g. antigens ultimately to be bound or adsorbed. The surface here encompasses all areas, i.e. besides the outer surface, also the inner surface of cavities (pores) in the particle. The functionalities may be directly or indirectly bound to the surface.

According to the present invention, the nanoparticle(s) comprise silicon dioxide ($SiO_2$), optional in mixture with another material. The material thus can also be admixed with further components, where silicon dioxide typically has the highest proportion in a multicomponent system. The nanoparticles of the invention can comprise at least 80% of silicon dioxide, preferably at least 90%.

In a particularly preferred embodiment of the nanoparticles according to the invention, the material comprises silicon dioxide which is essentially pure, i.e. only comprises the impurities to be expected in the course of the preparation process. In a more preferred embodiment of the invention, the nanoparticle material consists of silicon dioxide. Examples of other materials are metals, a metal chalcogenide, a magnetic material, a magnetic alloy, a semiconductor material, metal oxides, polymers, organosilanes, other ceramics or glass. The metal is selected from the group Au, Ag, Cu, Pt, Pd, Fe, Co, Gd, Ru, Rh and Zn, or any combination thereof.

In a further embodiment of the present invention the nanoparticles have a coating which comprises silicon dioxide. The core may comprise any other material such as metals, polymers or ferromagnetic metals such as $Fe_2O_3$ or $Fe_3O_4$. The core can even be devoid of silicon dioxide.

Silicon dioxide nanoparticles according to the invention have been described in, for example, WO 2010/006753 A2, which is expressly incorporated herein by reference.

The silicon dioxide nanoparticles can be prepared using, inter alia, the classical Stöber synthesis, in which monodisperse nanoscale silicon dioxide of defined size can be prepared by hydrolysis of tetraethoxysilane (TEOS) in aqueous-alcoholic-ammonia medium (J. Colloid Interface Sci. 1968, 26, 62).

The process of the preparation of silicon dioxide nanoparticles is described in detail in EP 0216 278 B1 and WO 2005/085135 A1, and consequently these documents are incorporated in their totality into the disclosure content of the present invention by way of reference. At least one amine is preferably used in the medium.

The silicon dioxide matrix of the nanoparticles according to the invention can be either porous or non-porous. The porosity is essentially dependent on the production process. In the synthesis in accordance with EP 0 216 278 B1, non-porous particles, in particular, are obtained.

According to the present invention the nanoparticles contain functional groups on their surface. These functional groups are capable to carry and/or stabilize both negative and positive charges. These charges belong the pharmaceutically acceptable compounds of the group of TLR agonists such as poly(I:C) and its derivatives or antigens or epitopes.

Preferred TLR agonists are TLR3 agonists selected from the group consisting of poly(I:C) (dsRNA, TLR3 agonist, as well as RIG-I agonist), polyI:poly$C_{12}$U (dsRNA, TLR3 agonist, trade name Ampligen®, INN: Rintatolimod) and NAB2 (Nucleic acid band 2, dsRNA isolated from yeast and identified as an agonist of the pattern-recognition receptors TLR3 and MDA-5) . More preferred is poly(I:C) LMW (Low Molecular Weight Poly(I:C) with an average size from 0.2 kb to 1 kb), poly(I:C) HMW (High Molecular Weight Poly(I:C) with an average size from 1.5 kb to 8 kb), polyI:poly$C_{12}$U or poly-ICLC. Most preferred is poly(I:C) LMW.

Functional groups which are capable to carry and/or stabilize both negative and positive charges are for example -SH, -COOH, $-NH_2$, -guanidino-group ($-NHC(=NH))NH_2$), $-PO_3H_2$, $-PO_2CH_3H$, $-SO_3H$, -OH, $-NR_3^+X^-$- Preferred functional groups are -COOH, -guanidino-group ($-NHC(=NH))NH_2$) and $-NH_2$. The functional groups may also be present in their salt form.

Compositions according to the invention comprise nanoparticles which have a surface loading density up to 0.5, preferably between 0.01 and 0.5, more preferred between 0.03 and 0.4, most preferred between 0.05 to 0.3, in

relation to the total number of the pharmaceutically acceptable compounds with regard to the surface of the nanoparticle in $nm^2$ [$molecules/nm^2$].

This surface loading density is calculated by assuming a perfect sphere and by the molar loading per particle. For example: a spherical particle with a diameter of 25 nm has a surface of 1964 $nm^2$ ($A=\pi*d^2$) and a weight of 1.64E-8 ng (with an assumed density of 2000 $kg/m^3$ for amorphous silica). The loading of such a particle with 5 % by weight with the peptide KKKV-Cit-YMLDLQPET (M=1,910.31 g/mol) equals to 4.283E-22 mol. This value multiplied by the Avogadro constant results in 258 single molecules of KKKV-Cit-YMLDLQPET attached to one particle with 1964 $nm^2$. This corresponds to a surface loading density of 0.13 $molecules/nm^2$.

The compositions according to the invention are formulated to have a pH between 6.0 and 8.0, preferably between 6.5 and 7.8 and more preferably between 6.8 and 7.5, even more preferred between 7.2 and 7.4. The pH of a composition can be maintained by the use of a buffer such as acetate, citrate, phosphate, succinate, TRIS (tris(hydroxymethyl)aminomethane) or histidine, typically employed in the range from about 1 mM to 50 mM. The pH of compositions can otherwise be adjusted by using physiologically acceptable acids or bases.

In one embodiment of the present invention the functional groups are $-PO_3H$ groups. Phosphorylated nanoparticles according to the invention could be for example prepared by reaction of (diethylphosphatoethyl)triethoxysilane with silica nanoparticles under addition of ammonia.

In a preferred embodiment of the present invention the functional groups are connected to the nanoparticle via a linker L. The linker can be connected to the nanoparticles e.g. by way of a covalent or adsorptive bond.

In a preferred embodiment of the present invention the linker compound L comprises at least one carboxyl (-COOH) or carboxylate ($-COO^-$) group as functional group.

In a more preferred embodiment such linker further comprises at least one guanidino-group ($-NHC(=NH)NH_2$ or $-NHC(=NH_2^+)NH_2$) or amino-group ($-NH_2$ or $-NH_3^+$) as functional group.

In a most preferred embodiment of the present invention the linker compound L contains at least a structural unit of the general formula (I)

$$*-(-O)_3Si-(CH_2)_nCH(COOX)-(CH_2)_p-C(O)-NH-CH(COOX)-(CH_2)_q-Y \quad (I)$$

wherein

X is independently from each other H or a negative charge,
Y is independently from each other $-NHC(=NH)NH_2$, $-NHC(=NH_2^+)NH_2$, $-NH_2$ or $-NH_3^+$,
n, p and q are independently from each other 0 or a number from 1 to 25; and
*- is the connection point to the nanoparticle.

In a preferred embodiment the linker compounds L contain at least a structural unit of formula (I) wherein n is 3, p is 1 and q is 4 and Y is independently from each other a $-NH_2$ or $-NH_3^+$ group.

In a particularly preferred embodiment the linker compounds L contain at least a structural unit of formula (I) wherein n is 3, p is 1 and q is 3 and Y is independently from each other a $-NHC(=NH)NH_2$ or $-NHC(=NH_2^+)NH_2$ group.

Another aspect of the present invention is to provide a process of preparation of nanoparticles according to the invention wherein

- in a first step (i) hydrolytic polycondensation of tetraalkoxysilanes and/or organotrialkoxysilanes in a medium which comprises water, at least one solubilizer and at least one amine or ammonia take place, where firstly a sol of primary particles is produced, and the resultant nanoparticles are subsequently brought to the desired particle size in a range from 5 to 150 nm in such a way that further nucleation is limited by continuous metering-in of corresponding silane in a controlled manner corresponding to the extent of reaction, and
- in a second step (ii) the nanoparticles from step (i) are reacted with [(3-triethoxysilyl)propyl]succinic anhydride, which in a simultaneous reaction forms an amide with L-arginine or L-lysine.

It is preferred to use in step (ii) of the process L-arginine.

In an alternative embodiment the linker compound L could be also obtained by the reaction of L-arginine with N-(3-triethoxysilylpropyl)maleimide. The reaction is carried out preferably at pH values above 8.

In one embodiment of the present invention it also possible to produce the linker compound L by reaction of [(3-triethoxysilyl)propyl]succinic anhydride with agmatine, histamine, cadaverine or spermidine.

A further embodiment of the present invention are nanoparticles comprising a silicon dioxide based surface with a linker compound L covalently or adsorptive bonded to it, wherein the Linker L contains at least one guanidino-group ($-NHC(=NH)NH_2$ or - $NHC(=NH_2^+)NH_2$) or amino-group ($-NH_2$ or $-NH_3^+$) as a functional group. In a preferred embodiment the Linker L contains at least one guanidino-group ($-NHC(=NH)NH_2$ or - $NHC(=NH_2^+)NH_2$) or amino-group

($-NH_2$ or $-NH_3^+$) and at least one carboxyl (-COOH) or carboxylate ($-COO^-$) group as a functional group. In a more preferred embodiment the Linker L contains at least one guanidino-group ($-NHC(=NH)NH_2$ or - $NHC(=NH_2^+)NH_2$) and at least one carboxyl (--COOH) or carboxylate ($-COO^-$) group as a functional group.

One embodiment of the nanoparticles according to the invention is illustrated in **Fig. 3.**

**Fig. 3** shows the schematic drawing of the cross section of a nanoparticle according to the invention. A represents the surface functionalization with the linker compound L, B represents the amorphous $SiO_2$ shell and C represents the core material, which could be void, water or any other material as well as amorphous $SiO_2$. The diameter d1 is between 0 and 149 nm and d2 is between 10 and 150 nm.

In one embodiment of the invention poly(I:C), any antigen or epitope are conjugated to the nanoparticle by adsorptive or covalent attachment. The adsorptive attachment is preferred.

A further object of the present invention are nanoparticles having silicon dioxide and functional groups on the surface and a particle size below 150 nm, comprising a linker L which is covalently or adsorptive bonded to it, wherein the Linker L comprises at least one guanidino-group ($-NHC(=NH)NH_2$ or $-NHC(=NH_2^+)NH_2$) or amino-group ($-NH_2$ or $-NH_3^+$) group as a functional group.

A main advantage of the adsorptive binding of the pharmaceutically acceptable compound is that, in contrast to most covalent conjugations, no by-products are formed or remain in the "reaction" mixture. In case of a covalent attachment it could become necessary to chemically modify the molecule which should be attached by adding reactive functional groups to the molecule. This is a fundamental intervention in the structure of the peptide/antigen and constitutes a complex chemical modification in case of RNA- or DNA-based antigens or adjuvants. In addition, a covalent linkage of a known compound that has already been approved by the drug authorities creates a new, independent substance (New Chemical Entity, NCE) which, for regulatory reasons, requires a new approval.

In a preferred embodiment the linker compound L according to formula (I) is able to carry and/or stabilize by way of adsorption pharmaceutically acceptable compounds which have positive or negative charges.

In a particularly preferred embodiment the linker compound L according to formula (I) is able to carry and/or stabilize by way of adsorption poly(I:C) which has negative charges.

In a more preferred embodiment the linker compound L according to formula (I) is able to carry and/or stabilize by way of adsorption pharmaceutically acceptable compounds which have phosphate or phosphonate groups.

It was surprisingly found that the electrostatic interaction between the arginine group of the linker compound L and the negative charge of the phosphate group of the pharmaceutically acceptable compound possesses a 'covalent-like' stability.

The term "attachment" here relates to any type of interaction between the surface functionality and the antigen, in particular covalent and non-covalent bonds, such as, for example, hydrophobic/hydrophilic interactions, van der Waals forces, ionic bonding, hydrogen bonds, ligand-receptor interactions, base pairing of nucleotides or interactions between epitope and antibody binding site.

In case where the pharmaceutically acceptable compound is hydrophobic, it is advantageous to increase the hydrophilicity thereof. In case of a protein or peptide this is possible e.g. by an N- or C-terminal extension with polar amino acids. In a preferred embodiment of the invention the protein or peptide is extended with one or more polar amino acids selected from the group comprising aspartic acid, glutamic acid, histidine, lysine, arginine, serine, threonine or tyrosine, preferably lysine, arginine, glutamic acid and aspartic acid more preferred lysine.

The present invention is therefore particularly directed to a tripartite bioconjugate which comprises an N- or C-terminal extension with polar amino acids for enhancing the solubility and which is connected to the linker compound L by adsorptive linkage, a linker unit U and a peptide which is illustrated in Fig. 4a.

A linker unit U according to the invention is for example used in antibody-drug conjugates (ADCs) which contain various types of linkers. There are three main types of chemically cleavable linkers: acid cleavable, reducible disulfides and those cleavable by exogenous stimuli.

Examples of ADCs are Gemtuzumab ozogamicin (Mylotarg® by Pfizer, linker is 4-(4-acetylphenoxy)butanoic acid), Inotuzumab ozogamicin (Besponsa® by Pfizer, linker is condensation product of 4-(4'-acetylphenoxy)-butanoic acid (AcBut) and 3-methyl-3-mercaptobutane hydrazide (known as dimethylhydrazide), Trastuzumab emtansin (Kadcyla® by Roche, linker is 4-[N-Maleimidomethyl]cyclohexan-1-carboxylate) or Brentuximab vedotin (also known as SGN-035; Adcetris® by Seattle Genetics Inc., linker is the dipeptide valine-citrulline).

In a preferred embodiment of the present invention the peptides comprise as linker unit U comprising an N-terminal extension with an enzymatic cathepsin B-cleavable linker (catB-cleav-linker) to ensure the release of the native unmodified antigen for MHC I or MHC II. The enzymatic catB-cleav-linker comprises one of the cathepsin B sensitive dipeptides Val-Cit, Phe-Cit, Leu-Cit, Ile-Cit, Trp-Cit, Phe-Lys, Ala-Lys or Val-Lys, preferred dipeptides are Val-Cit or Trp-Cit, more preferred is Val-Cit.

In one embodiment the compositions according to the invention comprise phosphorylated nanoparticles and one or more peptides which are conjugated to the nanoparticles by adsorptive attachment, wherein the peptide comprises Val-Cit or Trp-Cit as cathepsin B sensitive dipeptide.

**Fig. 4b** shows a preferred tripartite bioconjugate according to the invention. The peptide used in **Fig. 4b** is an extreme hydrophobic epitope derived from human NY-ESO-1. For the N-terminal extension the enzymatic (cathepsin B) cleavable peptidic linker Val-Cit was used to ensure the release of the native unmodified antigen for MHC I or MHC II. For enhancing the solubility and the electrostatic attraction to the nanoparticle the extension part "Lys-Lys-Lys" was used. Also possible is the extension for example with Lys-Lys-Lys-Asp or $Arg_6$.

In a preferred embodiment of the present invention the HPV16 $E7_{82-90}$ epitope LLMGTLGIV is enlarged on the N-terminal site with the enzymatic cleavable linker Val-Cit and the cationic solubilizing sequence Lys-Lys-Lys, leading to KKKV-Cit-LLMGTLGIV.

In another preferred embodiment of the present invention the HPV16 $E7_{11-19}$ epitope YMLDLQPET is enlarged on the N-terminal site with the enzymatic cleavable linker Trp-Cit and the cationic solubilizing sequence Lys-Lys-Lys leading to KKKW-Cit-YMLDLQPET.

A further embodiment is a composition according to the invention comprising $SiO_2$-nanoparticles having linker compounds L on the surface, wherein the linker compound L comprises at least one guanidino-group ($-NHC(=NH)NH_2$ or $-NHC(=NH_2^+)NH_2$) and at least one carboxyl (-COOH) or carboxylate ($-COO^-$) group as functional groups and one or more peptides or antigens which are conjugated to the linker compound L by adsorptive attachment, wherein the peptide or antigen comprises a linker unit U and a hydrophilic elongation with one or more amino acids.

A preferred embodiment is a composition according to the invention comprising $SiO_2$-nanoparticles having linker compounds L on the surface, wherein the linker compound L comprises at least one guanidino-group ($-NHC(=NH)NH_2$ or $-NHC(=NH_2^+)NH_2$) and at least one carboxyl (-COOH) or carboxylate ($-COO^-$) group as functional groups and one or more peptides or antigens which are conjugated to the linker compound L by adsorptive attachment, wherein the peptide or antigen comprises a cathepsin B sensitive dipeptide and a hydrophilic elongation with Lys-Lys-Lys.

A more preferred embodiment is a composition according to the invention comprising $SiO_2$-nanoparticles having linker compounds L on the surface, wherein the linker compound L comprises at least one guanidino-group (--NHC(=NH)$NH_2$ or - NHC(=$NH_2^+$)$NH_2$) and at least one carboxyl (-COOH) or carboxylate ($-COO^-$) group as functional groups and one or more peptides or antigens which are conjugated to the linker compound L by adsorptive attachment, wherein the peptide or antigen comprises Val-Cit or Trp-Cit as cathepsin B sensitive dipeptide and a hydrophilic elongation with Lys-Lys-Lys.

Another embodiment is a composition according to the invention comprising $SiO_2$-nanoparticles having linker compounds L on the surface, wherein the linker compound L comprises at least one guanidino-group $-HC(=NH)NH_2$ or $-NHC(=NH_2^+)NH_2$) and at least one carboxyl (-COOH) or carboxylate ($-COO^-$) group as functional groups and the model peptide SIINFEKL which is conjugated to the linker compound L by adsorptive attachment, wherein the peptide comprises Val-Cit or Trp-Cit as cathepsin B sensitive dipeptide and a hydrophilic elongation with Lys-Lys-Lys.

However, this hydrophilic modification of the peptide could lead to a stronger binding to MHC I i.e. are immunodominant, thereby displacing the target epitope and could provoke an immune response against an epitope not present on the tumor cells. To avoid this effect, there could be the need of a spacer between the hydrophilizing sub-molecule and the epitope.

An optional embodiment of the present invention is the use of so-called self-immolative spacers (PABC, p-aminobenzylcarbamate). Examples of such self-immolative spacers are compounds which comprise the structural unit of 4-aminobenzyl alcohol, 2-aminobenzyl alcohol or 4-hydroxybenzyl alcohol, 2-hydroxybenzyl alcohol (EP-A 0 648 503, WO 2007/031734 A1, WO 2015/162291 A1, US 6,180,095 B1, US 6,214,345 B1).

A self-immolative spacer is defined as a molecular section which is chemically linked to at least two further molecular sections such that when one of the bonds to the molecular sections is released, the remaining bonds are split and the previously attached molecules are released.

If a self-immolative spacer is used, it is preferred that the spacer is placed between enzymatic catB-cleav-linker and the peptide. As an example the following bioconjugate could be produced: ($Arg_6$)-(Val-Cit)-(PABC)-(SIINFEKL), wherein SIINFEKL is a model antigen.

It was surprisingly found that the enzymatic catB-cleav-linker systems can be used without the need of a self-immolative spacer.

The epitopes used for therapeutic vaccination typically consist of 8 to 11 amino acids (for MHC I) and a maximum of 25 amino acids for MHC II. For those epitopes there is no need for using a self-immolative spacer, because of the small size of these epitopes in comparison to much larger drugs, inhibiting an enzymatic cleavage.

An "antigen" according to the invention is a structure, which is capable of inducing a cellular or humoral immune response. Antigens are preferably proteinogenic, i.e. they are proteins, polypeptides, peptides or fragments thereof. In principle, they can have any size, origin and molecular weight. They contain at least one antigenic determinant or an antigenic epitope.

In an alternative embodiment the antigen is a nucleic acids per se or is encoded by a nucleic acid, which, after transport into the nucleus of antigen-presenting cells, are translated into the proteinogenic antigen which is then presented by MHC molecules.

The nucleic acids can be single- and double-stranded DNA or RNA or oligonucleotides. The nucleic acids may also be a constituent of complexes with lipids, carbohydrates, proteins or peptides.

A "peptide" according to the present invention is composed of any number of amino acids of any type, preferably naturally occurring amino acids, which preferably are linked by peptide bonds. In particular, a peptide comprises at least 3 amino acids, preferably at least 5, at least 7, at least 9, at least 12 or at least 15 amino acids. In a preferred embodiment the peptide does not exceed a length of 100 amino acids, more preferably, it does not exceed a length of 50 amino acids; even more preferably, it is not longer than 25 amino acids. In a most preferred embodiment the peptide has a length from 8 to 20 amino acids. The peptides can also exhibit posttranslational modifications, as e.g. phosphorylations, glycosylations, lipidations (like myristoylation or Palmitoylation), citrullinations, acetylations (of lysine), hydroxylations (of proline or lysine).

An "epitope" according to the invention, also known as antigenic determinant, is the part of an antigen that is recognized by the immune system, specifically by antibodies, B cells, or T cells. T cell epitopes are presented on the surface of an antigen-presenting cell, where they are bound to MHC molecules. In humans, professional antigen-presenting cells are specialized to present MHC class II peptides, whereas most nucleated somatic cells present MHC class I peptides. T cell epitopes presented by MHC class I molecules are typically peptides between 8 and 11 amino acids in length, whereas MHC class II molecules present longer peptides, 13-17 amino acids in length, and non-classical MHC molecules also present non-peptidic epitopes such as glycolipids.

The composition according to the invention may be used as immunostimulant. The immunostimulant according to the invention preferably contains a TLR3 agonist, more preferred poly(I:C) or any derivatives thereof as pharmaceutically acceptable compound. A further object of the present invention is an immunostimulant comprising the compositions according to the invention. The immunostimulant according to the invention may be administered to humans or animals, preferably livestock or companion animals.

In one embodiment of the present invention the compositions according to the invention is preferably administered before an immunosuppressive event. In case of an administration to animals, preferably cattle, such an immunosuppressive event, for example may be a result of increased stress or fatigue in the animals, which is caused by transport or when the stable is changed. A poor stable climate, a suboptimal supply of feed or water and an insufficient supply of colostrum for calves can also have an immunosuppressive effect. In case of an administration to humans, such an immunosuppressive event is for example an operative surgery.

Therefore, the compositions according to the invention are used for the prevention and/or treatment of Infectious Bovine Rinotracheitis (IBR) or Bovine Respiratory Disease (BRD), preferably BRD, which are often caused by a weakened immune system.

The compositions according to the invention are used for the perioperative immune stimulation in humans or for all stages of sepsis such as the Compensatory AntiInflammatory Response Syndrome (CARS).

In one embodiment of the present invention the compositions according to the invention are preferably administered to the patient prior to surgery.

An immunostimulant comprises one or more adjuvants and is used in adjuvant immunotherapy. As is well known in the art, adjuvants are agents that enhance immune responses. Adjuvants are well known in the art (e.g., see "Vaccine Design: The Subunit and Adjuvant Approach", Pharmaceutical Biotechnology, Volume 6, Eds. Powell and Newman, Plenum Press, New York and London, 1995).

Exemplary adjuvants include complete Freund's adjuvant (CFA), incomplete Freund's adjuvant (IFA), squalene, squalane and alum (aluminum hydroxide), which are materials well known in the art, and are available commercially from several sources. In certain embodiments, aluminum or calcium salts (e.g., hydroxide or phosphate salts) may be used as adjuvants. Alum (aluminum hydroxide) has been used in many existing vaccines.

In an alternatively preferred embodiment of the invention the compositions according to the invention comprise both, i.e. poly(I:C) and at least one antigen or epitope.

A more preferred embodiment is a composition according to the invention comprising $SiO_2$-nanoparticles having linker compounds L on the surface, wherein the linker compound L comprises at least one guanidino-group ($-NHC(=NH)NH_2$ or - $NHC(=NH_2^+)NH_2$) and at least one carboxyl (-COOH) or carboxylate ($-COO^-$) group as functional groups and poly(I:C) or any derivatives thereof and one or more antigens or epitopes which are conjugated to the linker compound L by adsorptive attachment, wherein the antigen or epitope comprises a linker unit U and a hydrophilic elongation with one or more amino acids.

The disclosed compositions and methods are applicable to a wide variety of antigens. In certain embodiments, the antigen is a protein (including recombinant proteins), polypeptide, or peptide (including synthetic peptides). In certain embodiments, the antigen is a lipid or a carbohydrate (polysaccharide). In certain embodiments, the antigen is a protein extract, cell (including tumor cell), or tissue.

In specific embodiments, antigens can be selected from the group consisting of the following:

(a) polypeptides suitable to induce an immune response against cancer cells;

(b) polypeptides suitable to induce an immune response against infectious diseases;
(c) polypeptides suitable to induce an immune response against allergens; and
(d) polypeptides suitable to induce an immune response in farm animals or pets.

In some embodiments, the antigen or antigenic determinant is one that is useful for the prevention of infectious disease. Such treatment will be useful to treat a wide variety of infectious diseases affecting a wide range of hosts, preferably human, but including cow, sheep, pig, dog, cat, and other mammalian species and non-mammalian species. Thus, antigens or antigenic determinants selected for the compositions will be well known to those in the medical art.

Appropriate antigens for use with compositions according to the invention may be derived from, but not limited to, pathogenic bacterial, fungal, or viral organisms, Corona virus (SARS-CoV-2), Influenza A virus (IAV), Influenza B virus, Bovine Respiratory Syncytial Virus (BRSV), Bovine Respiratory Coronavirus (BRCV), Human Papillomavirus (HPV), Human Immunodeficiency Virus (HIV), Bovine Herpesvirus-1 (BHV-1), Parainfluenza-type3-Virus (PI-3V), *Pasteurella multocida, Mannheimia haemolytica, Histophilus somni, Mycoplasma bovis.*

Examples of infectious disease include, but are not limited to, viral infectious diseases, such as COVID-19, Influenza (Flu), Acquired Immunodeficiency Syndrome (AIDS), Infectious Bovine Rinotracheitis (IBR), Bovine Respiratory Disease (BRD), SARS and malignancies caused by HPV.

Examples of cancers include, but are not limited to cervical cancer, anogential cancer, ovarian cancer, precursor lesions such as CIN (cervical intraepithelial neoplasia) or head & neck cancer.

Any antigen associated with any of the diseases or conditions provided herein can be used in the compositions and methods described herein. These include antigens associated with cancer, infections or infectious disease or degenerative or non-autoimmune disease. Preferred epitopes are those which are associated with a human papillomavirus infection (such as YMLDLQPET (HPV16 $E7_{11-19}$), FAFRDLCIVY (HPV16 $E6_{52-61}$), TIHDIILECV (HPV16 $E6_{29-38}$), RRFHNIAGHYR (HPV18 $E6_{125-135}$), KATLQDIVLHLE (HPV18 $E7_{5-16}$), HVDIRTLEDLLM (HPV16 $E7_{73-84}$), LEDLLMGTL (HPV16 $E7_{79-87}$), LLMGTLGIV (HPV16 $E7_{82-90}$) the cancer antigen *New York esophageal squamous cell carcinoma-1* (NY-ESO-1) and neoantigens as a class of tumor-specific antigens.

In another embodiment, antigens associated with infection or infectious diseases are associated with any of the infectious agents provided herein.

In one embodiment, the infectious agent is a virus of Papillomaviridae, Influenza virus or virus of the SARS family. In still another embodiment, the infectious agent is SARS-CoV-2 or Human papillomavirus, Influenza A virus.

In one embodiment the compositions according to the invention are used as vaccines for personalized cancer treatment.

The vaccines may be used as therapeutic vaccines or prophylactic vaccines, preferably for humans.

The compositions according to the invention are used as vaccines for the treatment of HPV positive humans or the treatment of HPV positive tumors.
A further object of the present invention is to provide a vaccine comprising compositions according to the invention.

Compositions according to the present invention are preferably stable dispersions.

The nanoparticles can be in dispersed form in any desired solvent, so long as the nanoparticles are neither chemically attacked nor physically modified by the solvent, and vice versa, so that the resultant nano-dispersion is stable, in particular pharmaceutically and physically stable. The dispersion is specifically characterized in that the nanoparticles are in monodisperse and non-aggregated form and have no tendency towards sedimentation, which results in sterile filterability.

Also object of the present invention is a lyophilisate comprising nanoparticles according to the invention. The lyophilisate may comprise additives for example polymers (e.g. polyethylene glycol, polyvinyl pyrrolidone, hydroxyethyl starch, dextran and ficoll) and sugars, (e.g. trehalose, lactose, sucrose, glucose, galactose, maltose, mannose and fructose), polyhydroxy alcohols (e.g. mannitol, sorbitol and inositol), amino acids (e.g. glycine, alanine, proline and lysine) and methylamines (e.g. trimethylamine-N- oxide, betaine and sarcosine). Lyophilisates according to the invention could be resuspended with sterile water before vaccination.

A pharmaceutical composition according to the present invention is any composition which can be employed in the prophylaxis, therapy, control or post-treatment of patients who exhibit, at least temporarily, a pathogenic modification of the overall condition or the condition of individual parts of the patient organism, in particular as a consequence of infectious diseases, septic shock, tumors, cancer, autoimmune diseases, allergies and chronic or acute inflammation processes. Thus, in particular, it is possible for the pharmaceutical composition in the sense of the invention to be a vaccine and/or an immunotherapeutic agent.

The compositions according to the invention may be formulated as pharmaceutical compositions that may be in the forms of solid or liquid compositions. Physiological saline solution or glycerol or glycols such as propylene glycol or polyethylene glycol may be included.

The compositions according to the present invention optionally may comprise other active ingredients or may comprise one or more of a pharmaceutically acceptable excipient, carrier, buffer, stabilizer, isotonic agent, preservative

or anti-oxidant or other materials well known to those skilled in the art. Such materials should be non-toxic and should not interfere with the efficacy of the pharmaceutically acceptable compound. The precise nature of the carrier or other material may depend on the route of administration, e.g. orally or parenterally.

For intravenous, cutaneous or subcutaneous injection, or injection at the site of affliction, the composition according to the present invention will be in the form of a parenterally acceptable aqueous solution which is pyrogen-free and has suitable pH, isotonicity and stability.

Preservatives are generally included in compositions according to the invention to retard microbial growth, extending the shelf life of the compositions and allowing multiple use packaging. Examples of preservatives include phenol, meta-cresol, benzyl alcohol, para-hydroxybenzoic acid and its esters, methyl paraben, propyl paraben, benzalkonium chloride, 1-thioglycerol and benzethonium chloride.

The nanoparticle-containing compositions of the invention may be administered to patients by any number of different routes, including enteral or parenteral routes. Parenteral administration of the pharmaceutical composition is preferred. Parenteral administration includes administration by the following routes: cutaneous or subcutaneous, nasal, vaginal, rectal, intramuscular, intraocular, transepithelial, intraperitoneal, intracardiac, intraosseous, intradermal, intrathecal, intraperitoneal, transdermal, transmucosal, and inhalational and topical (including dermal, ocular, rectal, nasal, vaginal, inhalation and aerosol), and rectal systemic routes. In a preferred embodiment, the pharmaceutical composition is for local (e.g., mucosa, skin) applications.

Administration be performed e.g. by injection, or ballistically using a delivery gun to accelerate their transdermal passage through the outer layer of the epidermis. The nanoparticles can then be taken up, e.g. by dendritic cells, which mature as they migrate through the lymphatic system, resulting in modulation of the immune response and vaccination against the epitopic peptide and/or the antigen from which the epitopic peptide was derived or of which it forms a part. The nanoparticles may also be delivered in aerosols. This is made possible by the small size of the nanoparticles.

Particularly preferred is the injection an intradermal, subcutaneous or intramuscular or injection. The administration can be carried out, for example, with the aid of so-called vaccination guns or by means of syringes. Mucosal administration, in particular mucosal vaccination, can be beneficial for cases were pathogens enter the body via the mucosal route. Mucosal delivery routes primarily include the oral and rectal route. Sometimes a pretreatment of the mucosa is necessary. It is also possible to prepare the substance as an aerosol, which is inhaled by the organism, preferably a human patient and taken up by the nasal and or bronchial mucosa. Other possible forms of mucosal administration are vaginal and rectal suppositories. These forms of administration are cost-effective (no consumables like syringes and needles), safe (very low risk of infection and operating errors), easy to apply and comprised with a high patients compliance (no needle fear). It also addresses the mucosal immune system (MALT) directly.

In one embodiment of the present invention the vaccines comprising the compositions according to the invention are used for a mucosal administration.

The exceptionally small size of the nanoparticles of the present invention is a great advantage for delivery to cells and tissues, as they can be taken up by cells even when linked to targeting or therapeutic molecules. Thus, the nanoparticles may be internalized by APCs, the epitopic peptides processed and presented via class I MHC and class II MHC.

## PARTICULARLY PREFERRED EMBODIMENTS OF THE INVENTION

1. Composition comprising nanoparticles which are loaded with pharmaceutically acceptable compounds comprising polyinosinic:polycytidylic acid, poly(I:C) or any derivatives thereof, having silicon dioxide and functional groups on the surface, wherein

- the functional groups are capable to carry and/or stabilize both negative and positive charges of the pharmaceutically acceptable compounds,
- the zeta potential of the composition has a value of at least $\pm$ 15 mV,
- the nanoparticles have a particle size below 150 nm, preferably 100 nm or less.

2. Composition according to embodiment 1, wherein the nanoparticles are loaded with pharmaceutically acceptable compounds comprising polyinosinic:polycytidylic acid, poly(I:C) or any derivatives thereof and an antigen or epitope.

3. Composition according to embodiment 1 or 2, wherein derivatives of poly(I:C) are poly(I:C) LMW (Low Molecular Weight Poly(I:C)) with an average size from 0.2 kb to 1 kb), poly(I:C) HMW (High Molecular Weight Poly(I:C)) with an average size from 1.5 kb to 8 kb), polyI:polyC$_{12}$U.

4. Composition according to any of embodiments 1 to 3, wherein the nanoparticles have a surface loading density up to 0.5, preferably between 0.01 and 0.5, more preferred between 0.03 and 0.4, most preferred between 0.05 to 0.3, in relation to the total number of the pharmaceutically acceptable compounds with regard to the surface of the nanoparticle in $nm^2$ [molecules/$nm^2$].

5. Composition according to any of embodiments 1 to 4, wherein the Polydispersity Index (PDI) of the composition is between 0 and 0.32, preferably between 0.1 and 0.3, more preferably between 0.1 and 0.2, most preferred less than 0.1.

6. Composition according to any of embodiments 1 to 5, wherein the pH value of the composition is between 6.0 and 8.0.

7. Composition according to any of the preceding embodiments, wherein the zeta potential of the composition has a value of at least $\pm$ 30 mV.

8. Composition according to any of the preceding embodiments, wherein the Z-average diameter of the nanoparticles is in the range of $\geq$5 and <150 nm, preferably $\geq$15 and $\leq$60nm, more preferably $\geq$20 and $\leq$50nm and still more preferably between 20 and 30nm.

9. Composition according to any of the preceding embodiments, wherein the net charge of the loaded nanoparticles is in total different to zero.

10. Composition according to any of the preceding embodiments, wherein the functional groups are connected to a linker L which is linked to the surface of the nanoparticles by way of a covalent or adsorptive bond.

11. Composition according to embodiment 10, wherein the linker compound L comprises at least one carboxyl (--COOH) or carboxylate ($-COO^-$) group as functional group.

12. Composition according to embodiment 10 or 11, wherein the linker compound L comprises at least one guanidino-group ($-NHC(=NH)NH_2$ or $-NHC(=NH_2^+)NH_2$) or amino-group ($-NH_2$ or $-NH_3^+$) group as a functional group.

13. Composition according to any of embodiments 10 to 12, wherein the linker L comprises at least one further functional group L', which is selected from the group -SH, -COOH, $-NH_2$, -guanidino-group ($-NHC(=NH))NH_2$), $-PO_3H_2$, $-PO_2CH_3H$, $-SO_3H$, -OH, - $NR_3^+X^-$.

14. Composition according to any of embodiments 10 to 13, wherein the linker compound L contains at least a structural unit of formula (I)

*-(-O)3Si-(CH2)n-CH(COOX)-(CH2)p-C(O)-NH-CH(COOX)-CH2)q-Y (I)

wherein

| | |
|---|---|
| X | is independently from each other H or a negative charge, |
| Y | is independently from each other $-NHC(=NH)NH_2$, $-NHC(=NH_2^+)NH_2$, $-NH_2$ or $-NH_3^+$, |
| n, p and q | are independently from each other 0 or a number from 1 to 25; and |
| *- | is the connection point to the nanoparticle. |

15. Composition according to any of the preceding embodiments, wherein the pharmaceutically acceptable compound is conjugated to the nanoparticle by adsorptive attachment.

16. Composition according to any of the preceding embodiments, wherein the antigen is a peptide antigen.

17. Composition according to embodiment 16, wherein the peptide antigen comprises 8 to 100 amino acids.

18. Composition according to embodiment 17, wherein the peptide antigens are MHC class I epitopes or MHC class II epitopes.

19. Composition according to embodiments 17 or 18, wherein the peptide antigen is N- or C-terminally extended

with polar amino acids.

20. Composition according to embodiment 19, wherein the polar amino acids are selected from the group aspartic acid, glutamic acid, histidine, lysine, arginine, serine, threonine, tyrosine.

21. Composition according to any of embodiments 17 to 20, wherein the peptide antigen comprise an N-terminal extension with an enzymatic cathepsin B-cleavable linker.

22. Composition according to embodiment 21, wherein the enzymatic cleavable linker comprises one of the cathepsin B sensitive dipeptides Val-Cit, Phe-Cit, Leu-Cit, Ile-Cit, Trp-Cit, Phe-Lys, Ala- Lys or Val-Lys.

23. Composition according to embodiments 21 or 22, wherein a spacer is placed between enzymatic cleavable linker and peptide.

24. Composition according to embodiment 23, wherein the spacer is a self-immolative spacer.

25. Composition according to embodiment 23 or 24, wherein the spacer comprises the structural unit of 4-aminobenzyl alcohol, 2-aminobenzyl alcohol or 4-hydroxybenzyl alcohol, 2-hydroxybenzyl alcohol.

26. Composition according to any of the preceding embodiments, wherein the nanoparticles comprise at least silicon dioxide ($SiO_2$), optional in mixture with another material.

27. Composition according to any of the preceding embodiments, wherein the nanoparticles consist of $SiO_2$.

28. Composition according to any of the preceding embodiments, wherein the compositions are stable dispersions.

29. Composition according to any of the preceding embodiments, wherein the pH value of the composition is between 7.2 and 7.4.

30. Composition according to any of the preceding embodiments, wherein the composition is isotonic.

31. Composition according to any of the preceding embodiments, wherein the composition contains further pharmaceutically acceptable additives.

32. Composition according to any of the preceding embodiments for use as vaccine.

33. Composition according to any of the preceding embodiments for use as immunostimulant.

34. Composition according to embodiment 33 for use for the activation of the immune system and as adjuvant.

35. Composition according to embodiment 33 for the use of the prevention and/or treatment of bovine respiratory disease (BRD).

36. Compositions according to embodiment 33 for perioperative immune stimulation in humans.

37. Compositions according to embodiment 33 for the treatment of all stages of sepsis.

38. Composition according to any of the preceding embodiments for personalized cancer treatment.

39. Composition according to embodiment 38 for the treatment of cervical cancer, anogential cancer, ovarian cancer, head & neck cancer.

40. Vaccine comprising compositions according to any of the preceding embodiments.

41. Immunostimulant comprising compositions according to any of the preceding embodiments.

## EXAMPLES

### EXAMPLE 1

**Preparation of monodisperse Silicon Dioxide Nanoparticles with 25 nm Diameter**

500 mL ethanol absolute were taken from a 500 mL graduated measuring cylinder into a 1000 mL glass bottle with screw cap. 358 mL sterile DI water was added and the mixture was well shaken.
69.6 mL tetraethylorthosilicate (TEOS) were added to the bottle. The bottle was tightly closed and well shaken. After 1 hour waiting time the clear colorless mixture got room temperature (22 °C). Then 9.5 mL ammonia water (25 %) was added quickly.
The bottle was shaken by hand very well for 10 seconds and stored at room temperature.
After 24 hours at room temperature additional 34.8 mL TEOS were added to the mixture (seed-growth process), in order to get a more narrow size distribution. After additional 24 hours at room temperature again 34.8 mL were added to the mixture (seed-growth process continued). After 24 hours at room temperature 50 μL of the reaction mixture were placed in a one-way PMMA cuvette (10 mm width) and diluted with 1.5 mL DI water (0.2 μm filtered). The cuvette was placed in a ZetaSizer Nano for particle size measurement (ZetaSizer Nano ZS, Malvern Instruments, UK).

Applied parameters:

| | |
|---|---|
| Measurement Angle: | 173° Backscatter (NIBS default) |
| Measurement Cell: | DTS1070 (used for particle size and zeta potential) |
| Refractive Index SiO2: | 1.460 |
| Absorption: | 0.010 |
| Dispersant: | water |
| Refractive Index: | 1.330 |
| Viscosity: | 0.8872 cP (= sample viscosity) |
| Temperature: | 25 °C |

>10 measurement runs for particle size

**Results:**

| | |
|---|---|
| Z-average (volume based): | 23.86 nm |
| PDI: | 0.108 |
| Intensity Peak: | 26.77 nm |
| pH: | 11.0 (freshly calibrated combination electrode) |

The nanoparticle suspension was transferred into a 2 liter round bottom flask and the ethanol as well as the ammonia gas was removed by a rotary evaporator with heated water bath. 400 mL sterile DI water was added in portions. The volume was reduced down to 198.15 g.
The solid content (pure nano dispersed silicon dioxide) of the suspension was determined in triplicate via vaporization of 250 μL and weigh out the residue.

**Result:**

| | |
|---|---|
| Solid content: | 163.3 mg/mL |
| pH: | 8.0 (freshly calibrated combination electrode) |

The distillation residue was diluted with 264.5 mL sterile DI water, in order to get a solid content of 70.0 mg/mL.

## EXAMPLE 2

### Functionalization of the Nanoparticles with L-arginine to get Arginylated Silica Nanoparticles ($SiO_2$-Arg)

462.65 g of the nanoparticle suspension obtained in Example 1 were placed into a 500 mL glass bottle. A magnetic stir was added and the bottle was placed onto a magnetic stirrer. 17.35 g L(+)-Arginine (CAS No. 74-79-3, PanReac AppliChem, Ph. Eur., USP. product code A1345.0500) were added to the nanoparticle suspension. When the arginine was completely dissolved a pH value of 10.8 was obtained, then 5.34 mL 3-(Triethoxysilyl)propylsuccinic anhydride (CAS No. 93642-68-3, Gelest Corp. product code: SIT8192.6) were slowly added at room temperature. The reaction mixture got very turbid but cleared up within about 30 minutes. During this time two reactions took place simultaneously:
The ethoxy groups of the silane got hydrolyzed, due to the high pH value. The created silanol groups precipitated onto the silica nanoparticles building a surface coating on top of the nanoparticles. In parallel the amino group of the excess arginine reacts with the succininc anhydride group, forming an amide bond.
The pH value dropped to 9.8, due to the consumption of arginine and mainly due to the formation of a carboxylic group, by opening the cyclic anhydride.

After 24 hours of stirring the suspension was transferred into 20 falcons tubes with a 100 kDa membrane (Pall Corp. Macrosep® Advance, product code MAP100C38). The nanoparticle suspension was centrifuged for 10 minutes at 4,000 rpm (= 2,737 g at the used centrifuge) to a volume at least 5 less than the starting volume. After centrifugation the volume in the falcon tubes was restored with sterile DI water and the centrifugation was repeated 5 times. The centrifugation step is necessary to remove excess arginine and possible unbound reaction products.

After centrifugation the solid content of the collected supernatants was determined in triplicate. The method was the same as in Example 1.

**Result:**

| | |
|---|---|
| Yield: | 178.3 g |
| Solid content: | 97.16 mg/mL |

The nanoparticle suspension was diluted with 168.17 mL sterile DI water to get a final concentration of 50.0 mg/mL.

## EXAMPLE 3

### Synthesis of Phosphorylated Silica Nanoparticles with 25 nm Diameter

200 mL ethanol absolute were taken from a 250 mL graduated measuring cylinder into a 500 mL pressure-resistant glass bottle with screw cap 143.5 mL sterile DI water were added and the mixture was well shaken. 27.85 mL tetraethylorthosilicate (TEOS) were added to the bottle. The bottle was tightly closed and well shaken.
After 1 hour waiting time the clear colorless mixture got room temperature (22 °C). Then 3.95 mL ammonia water (25 %) was added quickly. The bottle was shaken by hand very well for 10 seconds and stored at room temperature. After 24 hours at room temperature additional 1.5 mL (Diethylphosphatoethyl)triethoxysilane (CAS No. 757-44-8, Gelest Corp. product code SID3412.0) were added to the mixture at room temperature. 10 mL ammonia water (25 %) was added too and the reaction mixture was placed in a water bath at 85 °C for 24 hours.
At this temperature two reactions took place in parallel: The ethoxy groups of the silane got hydrolyzed, due to the high pH value. The silanol groups precipitated onto the silica nanoparticles building a surface coating on top of the nanoparticles. In parallel, but much slower, the phosphoric acid ester got hydrolyzed to yield into the corresponding acid with ammonia as the counter ion. 50 μL of the reaction mixture were placed in a one-way PMMA cuvette (10 mm width) and diluted with 1.5 mL DI water (0.2 μm filtered). The cuvette was placed in a ZetaSizer Nano for particle size measurement.

**Results:**

| | |
|---|---|
| Z-average (volume based): | 21.3 nm |
| PDI: | 0.144 |
| Intensity Peak: | 27.7 nm |

The nanoparticle suspension was transferred into a 500 mL round bottom flask and the ethanol as well as the ammonia gas was removed by a rotary evaporator with heated water bath. 200 mL sterile DI water was added in portions. The volume was reduced down to 120 mL. This volume was placed into 6 falcon tubes with a 100 kDa membrane (Pall Corp. Macrosep® Advance, product code MAP100C38). The nanoparticle suspension was centrifuged for 10 minutes at 4,000 rpm (= 2,737 g at the used centrifuge) to a volume at least 80 less than the starting volume. After centrifugation the volume in the falcon tubes was restored with sterile DI water and the centrifugation was repeated 5 times. The centrifugation step is necessary to remove excess possible unbound reaction products. After centrifugation the solid content of the collected supernatants was determined in triplicate. The method was the same as in Example 1.

**Result:**

| | |
|---|---|
| Yield: | 83.3 g |
| Solid content: | 104.0 mg/mL |

The nanoparticle suspension was diluted with 86.6 mL sterile DI water to get a final concentration of 50.0 mg/mL.

<u>Reaction scheme 1:</u>

Example 1 = a) and Example 2 = b)

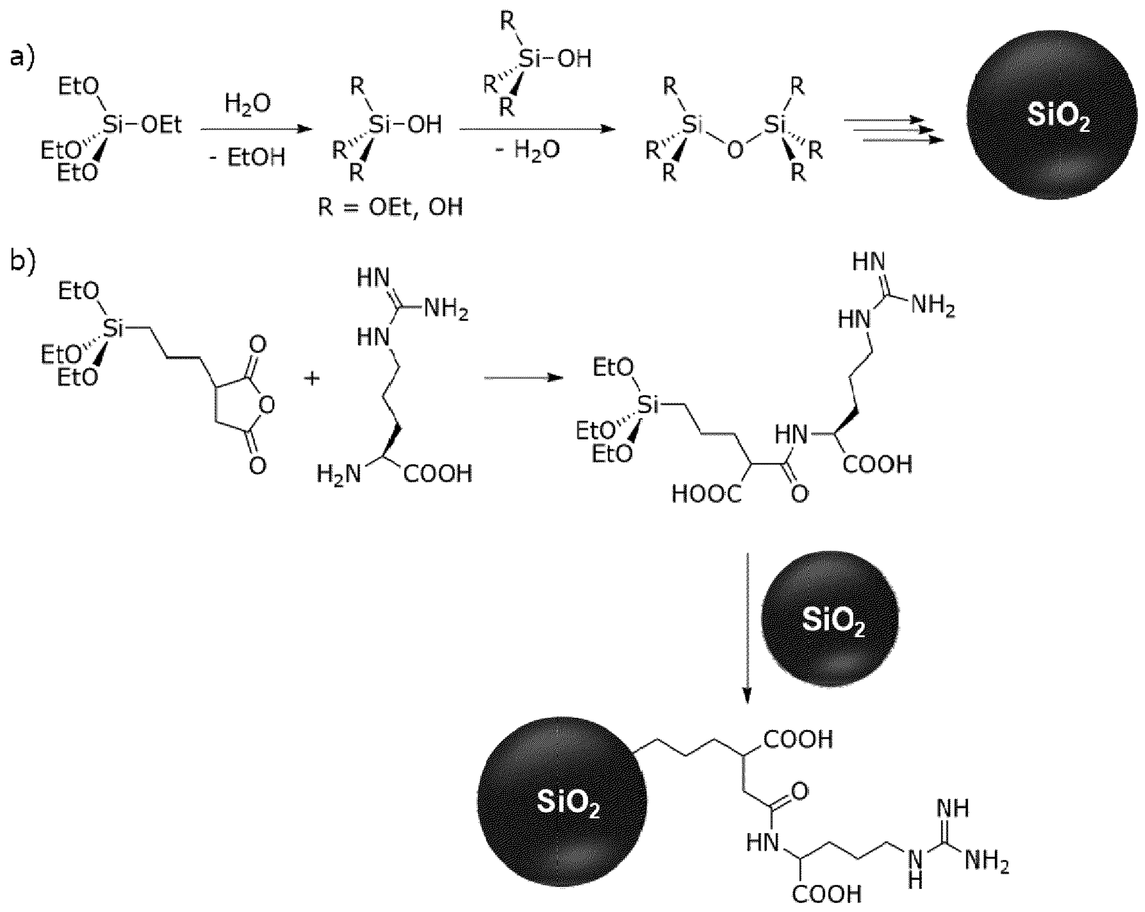

Reaction scheme 2:

Example 3

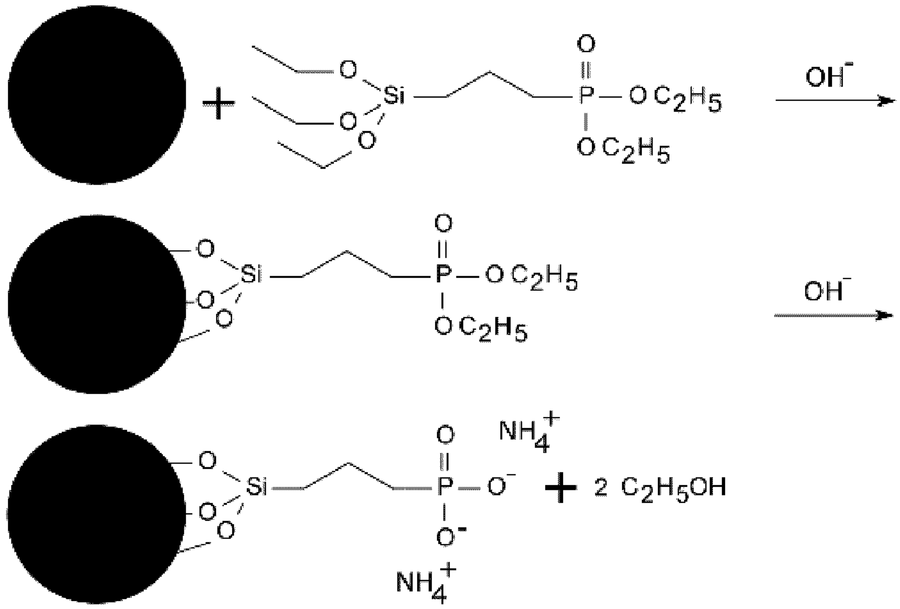


20

**EXAMPLE 4**

**Preparation of poly(I:C)@ $SiO_2$-Arg by adsorptive binding of poly(I:C)**

A mixture of 200 mL ethanol, 143.5 mL sterile de-ionized water and 27.85 mL tetraethyl orthosilicate (TEOS) was prepared. 3.70 mL ammonia, 25 % ($NH_3$ in water) were added at room temperature. The reaction mixture was mixed vigorously by shaking for 10 seconds and left at room temperature for 24 hours without stirring. The next day another portion of 27.85 mL tetraethyl orthosilicate (TEOS) was added to the mixture. From the resulting mixture 50 $\mu$L were removed and measured by means of dynamic light scattering (DLS) on a ZetaSizer Nano ZS (Malvern). The following results were obtained:

| | |
|---|---|
| Z-average: | 25.53 nm |
| mean: | 20.36 nm |
| polydispersity index (PDI): | 0.136 |

A portion of 100 ml of the silicon dioxide particles produced before were subsequently concentrated to a volume of about 30 ml on a rotary evaporator and filled up again to 100 ml. This procedure was repeated three times to remove the ethanol and ammonia from the reaction solution. The resulting suspension was washed five times over a 100 kDa membrane with sterile deionized water. After the last washing step, the solids content of the suspension was determined gravimetrically with 7.9% $SiO_2$ and the suspension was adjusted to a solids content of 5.0% by adding the calculated amount of water.

500 mg L-arginine (CAS number 74-79-3; company abcr GmbH, Germany) (= 2.87 mmol) was added to 15 ml of the silicon dioxide particles produced in Example 1 (750 mg $SiO_2$) and stirred while the arginine was completely dissolved. A clear particle suspension was obtained and to this suspension 127 $\mu$L (3-triethoxysilylpropyl) succinic anhydride (CAS number: 93642-68-3) (= 0.45 mmol) were slowly added while stirring at room temperature.

2.00 ml of the "argininylated" silica nanoparticles obtained above were mixed with 3.00 ml of a low molecular weight solution of poly(I:C) (poly(I:C)-LMW from InvivoGen, Toulouse, France with a size of 0.2 to 1 kb, CAS number 31852-29-6) with a concentration of 0.833 mg poly(I:C)/mL. After thoroughly mixing with a vortex mixer for 15 seconds and after one hour, the clear suspension was mixed with 250 mg of glucose. The formulation comprises:

| | |
|---|---|
| 100 mg | "argininylated" silica nanoparticles, $SiO_2$-Arg (c=20 mg/mL) |
| 2.5 mg | poly(I:C)-LMW (c = 0,5 mg/mL) |
| 250 mg | glucose (c = 50 mg/mL) (for isotonization of the formulation) |

The mixture was then filled into three sterile 2.0 mL HDPE vials using a 0.2 μm sterile filter. The obtained suspension is clear and completely transparent.
The poly(I:C) loaded particles pass easily a sterile filter. Two types of filters were used: Pall Life Sciences, Acrodisc Supor® Membrane (low protein binding) 0.2 μm, cat. no. PN4602 and VWR 0.2 μm Cellulose Acetate Membrane 0.2 μm, cat. no. 514-0061.
Even after adding larger amounts of poly(I:C), the suspension remains clear and completely transparent.

**EXAMPLE 5**

**Peptide Loading Capacity of $SiO_2$-Arg Nanoparticles**

In a peptide loading experiment 100 μL silica nanoparticles with a diameter of 25 nm (Z-average), a solid content of 20 mg/mL and an arginylated surface were loaded with different amounts of the model peptide KKKW-Cit-SIINFEKL. To simulate physiological conditions sodium chloride (NaCl) was added to get an isotonic suspension (0.9 % NaCl). Up to 10 % of peptide was added to the nanoparticles. KKKW-Cit-SIINFEKL has an iso-electric point of pH 10.24, indicating cationic properties determined by 4 basic amino acids (Lysine) and 1 acidic amino acid (Glutamic acid).
50 μL of the corresponding particle-peptide-NaCl mixture was diluted with 1.5 mL sterile filtered de-ionized water and measured via dynamic light scattering (DLS) in a ZetaSizer Nano (Malvern Instruments, UK). For every sample # 1 to 10 100 μL $SiO_2$-Arg stock solution was used. The obtained results are listed in Table 2.

Table 2: Results of the dynamic light scattering

| # | [mg/mL] $SiO_2$-Arg | [mg] peptide | [%] peptide | [μL] Peptide stock[2] | [mg] NaCl | [μL] NaCl stock[3] | z average [nm] | PDI |
|---|---|---|---|---|---|---|---|---|
| 0 | 20 | 0.00 | 0.00 | 0.0 | 0.90 | 9.00 | 23.62 | 0.07 |
| 1 | 20 | 0.02 | 1.00 | 5.1 | 0.95 | 9.45 | 24.00 | 0.10 |
| 2 | 20 | 0.04 | 2.00 | 10.2 | 0.99 | 9.92 | 24.48 | 0.10 |
| 3 | 20 | 0.06 | 3.00 | 15.5 | 1.04 | 10.39 | 26.03 | 0.14 |
| 4 | 20 | 0.08 | 4.00 | 20.8 | 1.09 | 10.87 | 26.91 | 0.14 |
| 5 | 20 | 0.11 | 5.00 | 26.3 | 1.14 | 11.37 | 28.,70 | 0.18 |
| 6 | 20 | 0.13 | 6.00 | 31.9 | 1.19 | 11.87 | 59.09 | 0.27 |
| 7 | 20 | 0.15 | 7.00 | 37.6 | 1.24 | 12.39 | 44.58 | 0.20 |
| 8 | 20 | 0.17 | 8.00 | 43.5 | 1.29 | 12.91 | 440.5 | 0.58 |
| 9 | 20 | 0.20 | 9.00 | 49.5 | 1.35 | 13.45 | 1074 | 0.31 |
| 10 | 20 | 0.22 | 10.00 | 55.6 | 1.40 | 14.00 | 2753 | 0.21 |

[1]Stock solution $SiO_2$-Arg: 20 mg/mL $SiO_2$-Arg
[2]Stock solution Peptide: 4 mg/mL Peptide (KKKW-Cit-SIINFEKL)
[3]Stock solution NaCl: 100 mg/mL NaCl

**Fig. 5a** illustrates the Z average versus peptide concentration and **Fig. 5b** the PDI versus peptide concentration.
As shown in **Fig. 5a** and **5b,** up to 5 % by weight of this peptide can be added to the nanoparticles, to keep a stable suspension without agglomeration or precipitation. Compared to bare particles the diameter is increasing slightly (+5 nm) and the PDI indicates a still quite narrow particle size distribution. This optical clear suspension is still passing a sterile filter.
The peptide loaded particles pass easily a sterile filter. Two types of filters were used: Pall Life Sciences, Acrodisc Supor® Membrane (low protein binding) 0.2 μm, cat. no. PN4602 and VWR 0.2 μm Cellulose Acetate Membrane 0.2 μm, cat. no. 514-0061.

With increased peptide loading the particle size increases, indicating the adsorptive peptide binding to the silica nanoparticles surface. At higher peptide concentrations - in the above example 6 % - the nano-suspension is collapsing, indicated by clouding and a measureable increased particle size due to agglomeration. These suspensions do not pass a sterile filter and are very unfavorable for parenteral administration.

## EXAMPLE 6

### Poly(I:C) Loading Capacity of $SiO_2$-Arg Nanoparticles

In a loading experiment 50 μL silica nanoparticles with a diameter of 25 nm (Z average), a solid content of 20 mg/mL and an arginylated surface were loaded with different amounts of poly(I:C) solution in RNase/DNase-free water by Gibco™. The poly(I:C) (LMW) was obtained from Invivogen Europe (cat. no. tlrl-picw). To simulate physiological conditions sodium chloride (NaCl) was added to get an isotonic suspension (0.9 % NaCl). Up to 8 % of poly(I:C) were added to the nanoparticles. The colloid remained stable: no precipitation or clouding was observable. The particle sizes and distributions, as well as the zeta potentials confirm the visual observations.

For the Zeta potential was measured according to the Smoluchowski calculation model under the following conditions:

Measurement Temp.: 25 °C
Equilibration Time: 30 s
10 to 100 runs per measurement, 5 measurements per sample (Zeta potential = mean of 5 measurements)

Table 3: Results of the dynamic light scattering

| [mg] poly(I:C) | [μL] poly(I:C ) stock | Vol [μL] | [mg] NaCl | [μL] NaCl stock | Z ave [nm] | PDI | Peak [nm] | int. Peak [nm] | Zeta potential |
|---|---|---|---|---|---|---|---|---|---|
| - | 50 | - | 0.45 | 4.50 | 33.83 | 0.557 | 12.69 | *) | - |
| 0.00 (0 %) | - | 50.0 | 0.45 | 4.50 | 22.79 | 0.077 | 20.23 | 24.47 | -27.4 |
| 0.01 (1 %) | 4.0 | 54.0 | 0.49 | 4.86 | 22.92 | 0.067 | 21.42 | 24.25 | -28.1 |
| 0.02 (2 %) | 8.2 | 58.2 | 0.52 | 5.23 | 23.12 | 0.075 | 19.96 | 23.97 | -28.7 |
| 0.04 (4 %) | 16.7 | 66.7 | 0.60 | 6.00 | 23.35 | 0.075 | 20.04 | 25.23 | -34.2 |
| 0.06 (6 %) | 25.5 | 75.5 | 0.68 | 6.80 | 23.45 | 0.08 | 19.96 | 34.93 | -36.2 |
| 0.09 (8 %) | 34.8 | 84.8 | 0.76 | 7.63 | 23.97 | 0.134 | **) | 32.15 | -37.2 |
| *) two peaks: 27.38 nm (67.8 %) and 362.6 nm (32.2 %)<br>**) two peaks: 7.691 nm (22.9 %) and 18.4 nm (77.1 %) | | | | | | | | | |

Up to a loading of 6 % poly(I:C) LMW the measured Z average values increases due to increase of particle diameter by adsorbed poly(I:C) **(Fig. 6b).** Also the low PDI (<0.1) indicates a smooth particle loading **(Fig. 6a).** At 8 % loading two peaks (at 7.691 and 18.4 nm) appear, also a strong peak broadening of the peak in direction to lower diameter values happens and the PDI value is getting worth **(Fig. 7d).** These data indicate particle "overloading": unbound poly(I:C) generates an additional peak, resp. broadens the measurement peak.

**Figures 7a** to **7d** show the Number % in a display of a ZetaSizer. The Number % is the representation of the particle distribution according to its percentage frequency.

**Fig. 7a:** Poly(I:C) LMW without nanoparticles
**Fig. 7b:** $SiO_2$-Arg nanoparticles without poly(I:C)
**Fig. 7c:** $SiO_2$-Arg nanoparticles loaded with 6 % poly(I:C) by weight

**Fig. 7d:** $SiO_2$-Arg nanoparticles loaded with 8 % poly(I:C) by weight

**EXAMPLE 7**

**Loading of $SiO_2$-Arg Nanoparticles with Peptides and Poly(I:C)**

In a peptide and poly(I:C) loading experiment 100 μL silica nanoparticles with a diameter of 25 nm (Z average), a solid content of 20 mg/mL and an arginylated surface were loaded with different amounts of the model peptide KKKW-Cit-SIINFEKL and with 50 μg poly IC (LMW) each (20 μL of a poly (I:C) stock solution, having a concentration of 2.5 mg/mL).
To simulate physiological conditions sodium chloride (NaCl) was added to get an isotonic suspension (0.9 % NaCl). Up to 4 % of peptide were added to the nanoparticles. The colloid remained stable: no precipitation or clouding was observable. The particle sizes and distributions, as well as the zeta potentials confirm the visual observations.

Table 4: Results of the dynamic light scattering

| [mg] pept. | [μL] pept. stock | Vol [μL] | [mg] NaCl | [μL] NaCl stoc k | [μg] Poly( I: C) | z ave [nm] | PDI | int. Peak [nm] | Peak [nm] | Zeta potential |
|---|---|---|---|---|---|---|---|---|---|---|
| 0.04 (2 %) | 10.2 | 110.2 | 0.99 | 9.92 | 50 | 24.09 | 0.172 | 24.57 | 19.48 | -37.1 |
| 0.06 (3 %) | 15.5 | 115.5 | 1.04 | 10.39 | 50 | 27.87 | 0.279 | 25.7 | 21.31 | -27.6 |
| 0.08 (4 %) | 20.8 | 120.8 | 1.09 | 10.87 | 50 | 28.47 | 0.305 | 25.41 | 20.76 | -25.9 |
| *(pept. = peptide) | | | | | | | | | | |

**EXAMPLE 8**

**Loading of $SiO_2$-Arg Nanoparticles with ssRNA (poly(U))**

In an ssRNA loading experiment 400 μL silica nanoparticles with a diameter of 25 nm (Z average), a solid content of 50 mg/mL and an arginylated surface (in total 20 mg silicon dioxide) were loaded with 500 μL poly(U) solution with a concentration of 1.0 mg/mL (in total 0.5 mg poly(U) and 100 μL sodium chloride (NaCl) with a concentration of 90 mg/mL was added to get an isotonic suspension (0.9 % NaCl). The loading of poly(U) on silica nanoparticles in this case is 2.44 % by weight. 50 μL of this sample were measure by DLS. The poly(U) was obtained from InvivoGen Europe, Toulouse, France (Cat. Code: tlrl-sspu).

Table 5: Results of the dynamic light scattering

| [mg] poly(U) | [mg] SiO2-Arg | [mg] NaCl | [μL] total volume | Z ave [nm] | PDI | Peak [nm] | int. Peak [nm] | Zeta potent ial |
|---|---|---|---|---|---|---|---|---|
| 0.5 (2 %) | 20.0 | 90 | 1000 | 23.19 | 0.065 | 20.21 | 24.93 | -31.6 |

**EXAMPLE 9**

**Loading of $SiO_2$-Arg Nanoparticles with unmethylated DNA (CpG ODN)**

In a DNA loading experiment 40 μL silica nanoparticles with a diameter of 25 nm (Z average), a solid content of 50 mg/mL and an arginylated surface (in total 2 mg silicon dioxide) were loaded with 50 μL ODN 2395 solution with a concentration of 1.0 mg/mL (in total 0.5 mg ODN 2395 and 10 μL sodium chloride (NaCl) with a concentration of 90 mg/mL was added to get an isotonic suspension (0.9 % NaCl). The loading of on silica nanoparticles in this case is 2.44 % by weight. 50 μL of this sample were measure by DLS.
ODN 2395 was obtained from InvivoGen, France (cat. code: tlrl-2395). It is a 22mer with the structure: 5'-tcgtcgtttt<u>cg-gcgc:gcgccg</u>-3' (bases are phosphorothioate (nuclease resistant), palindrome is underlined)

Table 6: Results of the dynamic light scattering

| [mg] CPG ODN | [mg] SiO2-Arg | [mg] NaCl | [μL] total volume | Z ave [nm] | PDI | Peak [nm] | int. Peak [nm] | Zeta potent ial |
|---|---|---|---|---|---|---|---|---|
| 0.05 (2.44 %) | 2.0 | 9 | 100 | 23.88 | 0.083 | 20.76 | 25.19 | -28.5 |

**EXAMPLE 10**

**Example for Solubility Enhancement of Epitopes**

On the N-terminal site of the enzymatically cleaved linker the addition of polar amino acids can be used to enhance the solubility of the whole peptide. The HLA-A:02 immunogenic HPV 16 E782-90 epitope LLMGTLGIV for example is extremely nonpolar and has a very bad solubility in water. The use in a human vaccine is difficult. In animal studies researchers dissolve it in DMSO. The solubility after e.g. subcutaneous injection is questionable: dilution might result in precipitation of large peptide particles, which is very unfavorable for transport to lymphnodes.
According to the invention the epitope LLMGTLGIV was enlarged on the N-terminal site with the enzymatic cleavable linker Val-Cit and the cationic solubilizing sequence Lys-Lys-Lys, leading to

**KKKV-Cit-LLMGTLGIV.**

The complete synthesis was done by automated microwave supported solid phase peptide synthesis (SPPS) in one run.
This peptide has an excellent solubility in water. After endosomal and/or cytosolic cleavage by cathepsin B the native HPV 16 E782-90 is released. Also the remaining KKKV-Cit is not immunogenic, due to the fact it's too short to be presented at any MHC I or MHC II.

**EXAMPLE 11**

**Human TLR-induced Cytokines in the Primary Human Macrophage Model (THP-1)**

Production of cytokines after stimulation of differentiated THP-1 cells with poly(I:C), $SiO_2$-Arg and poly(I:C)@ $SiO_2$-Arg were performed with Multi-Analyte ELISArray from Qiagen. Nanoparticle diameter is 22.1 nm (Z-average), PDI 0.08.
All experiments were performed with low molecular weight poly(I:C)-LMW from InvivoGen Europe, Toulouse, France.
The screening of cytokines showed significant production of cytokines:
**Fig. 8:** Cytokine expression (arbitrary units) for different types of cytokines after 72 h stimulation
TNF-α, IL8 (CXCL8), MCP-1 (CCL2), RANTES (CCL5), IP-10 (CXCL10), MIG (CXCL9) were highly expressed after 72 h stimulation with poly(I:C) @$SiO_2$-Arg. High cytokine release of IL8 (CXCL8), MCP-1 (CCL2), RANTES (CCL5), IP-10 (CXCL10), MIG (CXCL9) was also noticed for $SiO_2$-Arg nanoparticles.
**Fig. 9:** Cytokine expression (arbitrary units) for different types of cytokines after 96 h stimulation
After 96 h stimulation with poly(I:C) @$SiO_2$-Arg, the expression rate of cytokines IL1-β, IL12, IL17A, TARC, IFN-α is increased. High cytokine release of IL8 (CXCL8), MCP-1 (CCL2), RANTES (CCL5), IP-10 (CXCL10), MIG (CXCL9) was also noticed for $SiO_2$-Arg nanoparticles.
To investigate whether the combination of the TLR3 agonist poly(I:C)-LMW and $SiO_2$-Arg is able to stimulate cytokine release, the differentiated human macrophage-like THP-1 cells were incubated with poly(I:C)-LMW adsorptively bound to $SiO_2$-Arg (poly(I:C)@ $SiO_2$-Arg) or with the individual compounds and then subjected to cytokine-specific enzyme-linked immunosorbent assay (ELISA). The supernatant of the THP-1 cells was analyzed for interleukin 8 (IL-8) and tumor necrosis factor α (TNF-α) at several time points **(Fig. 10).** IL-8 and TNF-α are important mediators of the innate immune system response, regulating the activity of various immune cells. The release of these two cytokines is proof of a successful stimulation of the immune system.
**Fig. 10a and 10b:** Cytokine release at different time points after stimulation of differentiated THP-1 cells with poly(I:C) [12.5 μg/ml], $SiO_2$-Arg [0.5 mg/ml] or the novel adjuvant (poly(I:C) [12.5 μg/ml] bounded on $SiO_2$-Arg [0.5 mg/ml]).
**Fig. 10a:** Quantification of IL-8 release was performed using ELISA MAXTMDeluxe Set Human IL-8 from BioLegend, USA.

**Fig. 10b:** Quantification of TNF-α release was performed using ELISA MAXTMDeluxe Set Human TNF-α from BioLegend, USA.

**EXAMPLE 12**

**Experiments in the influenza A model of the mouse**

For the determination of the immunostimulatory potency of TLR3 agonists, a suspension is prepared according to Example 6 was tested in an in vivo study together with other active compounds as immunostimulators with regard to its prophylactic effect against a five-fold LD50 dose of the influenza A virus PR8/34.

The following active compounds were tested:

- Placebo: glucose solution (5 %)
- free poly(I:C) LMW: Low Molecular Weight poly(I:C) comprises short strands of inosinic acid poly(I) homopolymer annealed to strands of cytidinic acid poly(C) homopolymer. The average size of poly(I:C) LMW is from 0.2 kb to 1 kb (InvivoGen, France) in glucose solution (5 %)
- ZelNate®: FDA approved immunostimulant that aids in the prophylaxis of Bovine Respiratory Disease (BRD) due to *Mannheimia haemolytica* (Bayer AG, Germany)
- Poly(I:C)@$SiO_2$-arginylated prepared according to Example 6
- Silicon nanoparticles unmodified with a diameter of 25 nm in 5 % glucose solution

Table 7: Overview of prophylaxis trials in mice, every group consists of ten mice

| Group No. | active compound | composition | H1N1-dose; admin. |
|---|---|---|---|
| A | Placebo | - | 50 $TCID_{50}$ (5x $LD_{50}$); i.n. |
| B | Zelnate® | 100 μL | 50 $TCID_{50}$ (5x $LD_{50}$); i.n. |
| C | poly(I:C) (LMW) | 50 μg in 100 μL | 50 $TCID_{50}$ (5x $LD_{50}$); i.n. |
| D | $SiO_2$ (25nm) | 2mg $SiO_2$; (unmodified) | 50 $TCID_{50}$ (5x $LD_{50}$); i.n. |
| E | poly(I:C)@$SiO_2$-Arg (25 nm) | 2 mg $SiO_2$-"argynilated" and 50 μg poly(I:C) (LMW) in 100 μL | 50 $TCID_{50}$ (5x $LD_{50}$); i.n. |

In all cases the injection volume was 100 μL.

Each animal group (A to E), consisting of ten C57BL/6 mice, was treated 24 hours before administration of the influenza A virus subcutaneously with the respective active compound or placebo. The virus was administered intranasally.

In this study the body weight was used as a reliable and easy-to-measure marker for the animal health. Sick animals eat less and lose weight very quickly. For ethical reasons, the study defined a body weight loss of 25%, based on the body weight on the day of the virus administration, as the termination criterion. In contrast, in many publications from older studies, the "termination criterion" is the death of the animals due to the viral disease.

In this study, the formulation of poly(I:C) prepared according to Example 6, which is adsorptively bound to silica nanoparticles with an "argininylated" surface, showed a surprisingly strong immunostimulatory effect, which resulted in a statistically significantly longer survival rate of the animals of **group E** and to a significantly less weight loss.

The free poly(I:C), which is not bound to nanoparticles **(group C),** with the same concentration of active ingredient, does not show any statistically significant improvement compared to the placebo group **(group A).** The unmodified silica nanoparticles **(group D)** also showed no statistically significant improvement.

From the comparative experiments above, it could be shown that a TLR3 agonist which is adsorptively bound to silica nanoparticles **(group E),** is clearly superior over the free TLR3 agonist **(group C)** which was applied in the same concentration and under identical test conditions. The immune booster ZelNate® **(group B)** also showed no significant improvement in this study compared to the placebo group **(group A).** This also applies to the unmodified silica nanoparticles of **group D,** where no significant effect could be observed **(Fig. 11a and 11b).**

**Fig. 12** shows the **clinical scores.** Data are presented as mean clinical score (maximum score = 5; death of

animals, abort of experiment) ± SEM (n=10) in relation to days after challenge.

**EXAMPLE 13**

**Generation of HPV16 $E7_{11-19}$ specific CD8 T cell immune responses in tumor-free A2.DR1 mice**

The immunogenicity of human HPV16 E6/E7-derived, HLA-A2-binding epitopes can only be studied in genetically modified mice. Therefore the in vivo studies were performed using the HLA-humanized A2.DR1 BL6 mice. A2.DR1 mice are a highly sophisticated mouse model since they underwent a multitude of genetic alterations to exhibit the HLA-A2+/HLA-DR1+, H-2-phenotype and shown to assemble functional $CD4^+$ and $CD8^+$ T cell responses against multiple epitopes restricted by HLA-A2 and HLA-DR1 *(*Pajot, A. et al., A mouse model of human adaptive immune functions: HLA-A2.1-/HLA-DR1-transgenic H-2 class I-/class II-knockout mice, European Journal of Immunology, 2004, Vol. 34, p. 3060-3069*)*.

The efficacy of various formulations was examined for assess their ability to induce high frequencies of epitope-specific $CD8^+$ T cells after three weekly, *subcutaneous* immunizations (Day 0: Prime, Day 7: Boost, Day 14: Boost, Day 21: Spleen Sampling).

Used substances:

- poly(I:C)-HMW: The average size of poly(I:C)-HMW is 1.5 to 8 kb, InvivoGen, France
- $SiO_2$-$PO_3H_2$ according to Example 3

• *Preparation of KKKW-Cit-$E7_{11-19}$ + poly(I:C)-HMW @* $SiO_2$*-Arg*:

2.72 mg (1.48 μmol) KKKW-Cit-$E7_{11-19}$ are dissolved in 592 μL DNase/RNase free distilled water under sterile conditions. The solution is added under vortexing to 2.37 ml of a 50 mg/mL stock solution of $SiO_2$-Arg. 296 mg solid glucose are added and the solution is mixed until the solid has completely dissolved. Finally, 2.96 mL poly(I:C)-HMW is added as a 1.0 mg/mL stock solution while the sample is vortexed. The vaccine is filtered through a 0.45 μm filter. 1.8 mL each are filled into 3 separate vials (1 vial per immunization) and stored at 4°C.

The influence of the N-terminal peptide elongation of the HPV16 $E7_{11-19}$ (=YMLDLQPET in one letter code for amino acids) as well as the influence of the nanoparticles surface modification were investigated. To evaluate the vaccine-induced HPV16 $E7_{11-19}$-specific $CD8^+$ T cells, the ex vivo restimulated splenocytes were assessed in an IFN-γ intracellular cytokine staining (ICS) followed by flow cytometry **(Fig. 13).**

**Fig. 13:**

Y-axis:

HPV-001: RW-Cit-$E7_{11-19}$ [50nmol] + poly(I:C)-HMW [50 μg]
HPV-002: RW-Cit-$E7_{11-19}$ [50nmol] + poly(I:C)-HMW [50 μg] @ $SiO_2$-Arg [2.25 mg]
HPV-003: RW-Cit-$E7_{11-19}$ [50nmol] + poly(I:C)-HMW [50 μg] @ $SiO_2$-$PO_3H_2$ [2.25 mg]
HPV-004: KKKW-Cit-$E7_{11-19}$[50nmol] + poly(I:C)-HMW [50 μg] @ $SiO_2$-Arg [2.25 mg]

X-axis:

Percentage of IFN-γ+-CD8+ T cells (taken from mouse spleens)

Frequency of IFN-γ positive $E7_{11-19}$ specific $CD8^+$ T cells after ex vivo stimulation of splenocytes with $E7_{11-19}$ in the presence of Golgi apparatus-transport-inhibitors. After subsequent IFN-γ ICS, IFN-γ positive $E7_{11-19}$ specific T cells were determined by flow cytometry. Data are represented as the mean +/- SEM. Each dot represents one mouse.

As shown in **Fig. 13,** three immunizations with nanoparticles conjugated to epitope were able to induce antigen-specific splenic IFN-$\gamma^+$ $CD8^+$ T cells at higher frequencies in comparison to free epitope injections. Furthermore, the direct comparison of two surface modifications of nanoparticles (HPV-002 vs. HPV-003) demonstrated a better performance when the epitope was conjugated to $SiO_2$-Arg (HPV-002).

Moreover, the influence of the N-terminal peptide elongation (HPV-002 vs. HPV-004) was analyzed, where the "KKKW-Cit-"modification (HPV-004) led to higher frequencies of epitope-specific $CD8^+$ T cells.

This experiment successfully demonstrated the ability of the HPV16 vaccines according to the invention to

induce a high frequency of epitope specific $CD8^+$ T cells. In summary, $SiO_2$-Arg as well as the "KKKW-Cit-" peptide elongation have shown their beneficial properties over $SiO_2$-$PO_3H_2$ nanoparticles and the "RW-Cit-" peptide elongation. Therefore, the combination of both was used for assessing therapeutic efficacy in a tumor study.

## EXAMPLE 14

### Therapeutic efficacy in the PAP-A2-HPV16 tumor model

The final study goal is the development of a therapeutic anti-HPV16 vaccine, which would be given to patients diagnosed with either a precursor lesion or an established cancer. Therefore, the ability of the novel vaccines was tested to induce control of tumor growth in a therapeutic vaccination experiment.

Immunization schedule for early therapeutic treatment study in the PAP-A2-HPV16 tumor model (Kruse, S. Therapeutic vaccination against HPV-positive tumors in a MHC-humanized mouse model, Ruperto Carola University Heidelberg, 2019, Dissertati*on;* Kruse et al., Therapeutic vaccination using minimal HPV16 epitopes in a novel MHC-humanized, Oncoimmunology, 2019, Vol. 8, 1, p. e1524694):

To evaluate the therapeutic efficacy of $SiO_2$-Arg conjugated with a HPV16 E7-derived epitope, the HPV16 $E6^+$/$E7^+$ PAP-A2 tumor model in HLA-humanized A2.DR1 BL6 mice was used. $1.5{\cdot}10^6$ PAP-A2 cells were injected subcutaneously, which should result in large tumors within 2 to 3 weeks. Starting with day 4 after tumor inoculation, the tumor-bearing mice were treated weekly (3 immunizations total, Prime-Boost-Boost) with the complete vaccine (HPV-008) or with the individual compounds as controls, until the ethical endpoint (tumor volume 1000 $mm^3$) was reached.

**Fig. 14** shows the survival rate of mice, either receiving the free antigen HPV16 E7 YMLDLQPET + poly(I:C) (HMW, high molecular weight), shown as "free antigen + TLR agonist" or KKKW-Cit-YMLDLQPET + poly(I:C) (HMW) both adsorptively bound to arginylated silica nanoparticles having a diameter of 23 nm, shown as "HPV16Nano".

As shown in **Fig. 14,** the treatment of tumor-bearing mice with KKKW-Cit-YMLDLQPET + poly(I:C) (HMW) both adsorptively bound to arginylated silica nanoparticles (HPV16Nano) resulted in complete tumor regression (CR) in 5 out of 9 mice with overall survival rate of 55%. In contrast, 90% of carrier control (free antigen + TLR3 agonist) mice had to be eliminated due to excessive tumor growth. **Fig. 15a** and **Fig. 15b** show the individual tumor growth of both groups.

Taking together the results from therapeutic vaccinations with single compounds and with $SiO_2$-Arg conjugated with epitope, it can be concluded that the best therapeutic anti-tumor results were achieved with the triple surface-arginylated nanoparticles conjugated with KKKW-Cit-YMLDLQPET vaccination.

## EXAMPLE 15

### Cross-Presentation Experiments

*MHC I cross presentation of* $OVA_{257-264}$ ***after intracellular processing in DC2.4 cells***

To demonstrate the functionality of the enzymatic cleavage (W-Cit) site attached to an immunogenic epitope, experiments on cross-presentation of the model antigen $OVA_{257-264}$ (=SIINFEKL in one letter code for amino acids) in murine dendritic cells (DC2.4 cells, immortalized murine dendritic cells) were performed. Therefore, the expression of SIINFEKL on the Major Histocompatibility Complex I (MHC I) after incubation with various OVA-derived constructs, with and without nanoparticles, was determined by antibody (25-D1.16, PE/Cy7 anti-mouse H-2Kb bound to SIINFEKL Antibody, Biolegend, Inc., USA) specific detection of the native epitope presented on MHC I. Free, not MHC I bound epitope or elongated/modified or other epitopes on MHC I are not recognized by the antibody, which is highly selective to SIINFEKL presented on the MHC class I molecule H-2-Kb.

In the experiment, the $OVA_{257-264}$ presentation efficacy after incubation of $5{\cdot}10^4$ DC2.4 cells with 5 $\mu$M solutions of full length protein (OVA = Ovalbumin), N- and C-terminal elongated epitope ($OVA_{247-264}A_5K$, a so called synthetic long peptide (SLP)), N-terminal elongated epitope with a Cathepsin B cleavable sequence (exemplary shown RW-Cit-$OVA_{257-264}$) or native epitope ($OVA_{257-264}$), each with and without nanoparticles, was compared. Surface phosphorylated silica nanoparticles ($SiO_2$-$PO_3H_2$) with an average diameter of 25 nm (comparable size to $SiO_2$-Arg used in other experiments) were used as carrier. After an incubation time of 6 h, the test substances are removed by washing with phosphate-buffered saline (PBS). In the next step, the cells were incubated with CD16/CD32 antibody in order to block the non-specific binding of the detection antibody to the Fc (Fragment, crystallizable) receptor of the cells. After incubation with 25-D1.16 antibody, the amount of cross-presented $OVA_{257-264}$ was quantified by flow cytometry.

**Fig. 16:** $OVA_{257-264}$ MHC I presentation after 6 h incubation of H2-Kb positive cells with 5 $\mu$M solution of native or elongated $OVA_{257-264}$ epitope or full length OVA protein with or without $SiO_2$-$PO_3H_2$. Quantification was carried out by flow cytometry after labeling with 25-D1.16 detection antibody.

As shown in **Fig. 16,** only the native epitope and the elongated epitope with an enzymatic cleavage site are

presented in significant amounts on the MHC. The uptake and thus the amount of presented epitope can be increased slightly by incubation with peptides adsorptively bound to nanoparticles. While the native epitope does not necessarily have to be internalized into the cell, since it can also be loaded exogenously onto the MHC molecule, the N-terminal elongated epitope must be internalized to release the native sequence. The detection of the native epitope on MHC I after incubation of the cells with RW-Cit-$OVA_{257-264}$ confirms a proof of the functionality of the enzymatic cleavage site.

**EXAMPLE 16**

**Stability Measurement in Human Serum**

HEK-Blue™ hTLR3 Cells are designed to measure the stimulation of human TLR3 by monitoring the activation of NF-kB. HEK-Blue™ hTLR3Cells were obtained by co-transfection of the hTLR3 gene and an optimized secreted embryonic alkaline phosphatase (SEAP) reporter gene placed under the control of an NF-kB and AP-1-inducible promoter intoHEK293 cells. Stimulation with a TLR3 ligand activates NF-kB and AP-1 which induce the production of SEAP. Levels of SEAP can be easily determined with HEK-Blue™ Detection, a cell culture medium that allows for real-time detection of SEAP. The hydrolysis of the substrate by SEAP produces a purple/blue color that can be easily detected with the naked eye or measured with a 96 well plate reader. (Invivogen, see https://www.invivogen.com/hek-blue-htlr3).

Poly(I:C) and poly(I:C)@$SiO_2$-Arg were exposed for 60 minutes at 37 °C to human serum (HS). The serum was used in concentrations of 5, 10 and 20 %. A serum concentration of 20 % corresponds quite well to the composition of peripheral lymph.

Poly(I:C) and poly(I:C)@$SiO_2$-Arg were added separately to human serum to get a concentration of 1 μg poly(I:C)/mL. In case of poly(I:C)@$SiO_2$-Arg the $SiO_2$ concentration was 40 μg/mL. The poly(I:C) payload at $SiO_2$-Arg in this case was about 2.5 %. After exposure to HS 20 μL of the medium were taken and added to 180 μL HEK-Blue™hTLR3 cells in HEK-Blue™ Detection medium. This mixture was incubated for 13 hours at 37 °C and the plate was analyzed in a 96 well plate reader (Tecan Reader, Type: Infinite M200 Pro).

The results are shown in **Fig. 17.**

The calculated half-life of free poly(I:C) in 20 % human serum under the chosen conditions is 24.2 minutes (18 % of initial concentration after 60 minutes). Half-life calculation:

$$t_{½} = \ln 2 / (\ln 100 / \ln 18) * 60 \qquad [min]$$

The half-life for poly(I:C)@$SiO_2$-Arg under the same conditions is calculated to 395 minutes (90 % of initial concentration after 60 minutes)

$$t_{½} = \ln 2 / (\ln 100 / \ln 90) * 60 \qquad [min]$$

So the attachment of poly(I:C) to arginylated silica nanoparticles increases the half-life by a factor of 16 (=395 / 24.2).

**EXAMPLE 17**

**Stability Measurement in Bovine Serum**

HEK-Blue™ hTLR3 Cells are designed to measure the stimulation of human TLR3 by human TLR3 by monitoring the activation of NF-kB. HEK-Blue™hTLR3Cells were obtained by co-transfection of the hTLR3 gene and an optimized secreted embryonic alkaline phosphatase (SEAP) reporter gene placed under the control of an NF-kB and AP-1-inducible promoter intoHEK293 cells. Stimulation with a TLR3 ligand activates NF-kB and AP-1 which induce the production of SEAP. Levels of SEAP can be easily determined with HEK-Blue™ Detection, a cell culture medium that allows for real-time detection of SEAP. The hydrolysis of the substrate by SEAP produces a purple/blue color that can be easily detected with the naked eye or measured with a 96 well plate reader (Invivogen).

Poly(I:C) and poly(I:C)@$SiO_2$-Arg were exposed for 60 minutes at 37 °C to fetal bovine serum (FBS). The serum was used in concentrations of 5, 10 and 20 %. A serum concentration of 20 % corresponds quite well to the composition of peripheral lymph.

Poly(I:C) and poly(I:C)@$SiO_2$-Arg were added separately to bovine serum to get a concentration of 1 μg poly(I:C)/mL. In case of poly(I:C)@$SiO_2$-Arg the $SiO_2$ concentration was 40 μg/mL. The poly(I:C) payload at $SiO_2$-Arg in this case was about 2.5 %. After exposure to FBS 20 μL of the medium were taken and added to 180 μL HEK-Blue™

hTLR3 cells in HEK-Blue™ Detection medium. This mixture was incubated for 13 hours at 37 °C and the plate was analyzed in a 96 well plate reader (Tecan Reader, Type: Infinite M200 Pro).

The results can be seen in **Fig. 18:**

While the free poly(I:C) shows clear signs of degradation at higher serum concentrations, poly(I:C)@$SiO_2$-Arg shows markedly higher stability. Half-life calculation for poly(I:C)@$SiO_2$-Arg is useless, due to a very low decay rate.

## Claims

1. Composition comprising nanoparticles which are loaded with pharmaceutically acceptable compounds comprising polyinosinic:polycytidylic acid, poly(I:C) or any derivatives thereof, having silicon dioxide and functional groups on the surface, wherein

   - the functional groups are capable to carry and/or stabilize both negative and positive charges of the pharmaceutically acceptable compounds,
   - the zeta potential of the composition has a value of at least ± 15 mV,
   - the nanoparticles have a particle size below 150 nm.

2. Composition according to claim 1, wherein the nanoparticles are loaded with pharmaceutically acceptable compounds comprising polyinosinic:polycytidylic acid, (poly(I:C)) or any derivatives thereof and an antigen or epitope.

3. Composition according to claim 1 or 2, wherein derivatives of poly(I:C) are poly(I:C) LMW (Low Molecular Weight Poly(I:C) with an average size from 0.2 kb to 1 kb), poly(I:C) HMW (High Molecular Weight Poly(I:C) with an average size from 1.5 kb to 8 kb), polyI:poly$C_{12}$U.

4. Composition according to any of claims 1 to 3, wherein the nanoparticles have a surface loading density up to 0.5, in relation to the total number of the pharmaceutically acceptable compounds with regard to the surface of the nanoparticle in $nm^2$ [molecules/$nm^2$].

5. Composition according to any of the preceding claims, wherein the Polydispersity Index (PDI) of the composition is between 0 and 0.32.

6. Composition according to any of the preceding claims, wherein the zeta potential of the composition has a value of

at least ± 30 mV.

**7.** Composition according to any of the preceding claims, wherein the net charge of the loaded nanoparticles in total is different to zero.

**8.** Composition according to any of the preceding claims, wherein the functional groups are connected to a linker L which is linked to the surface of the nanoparticles by way of a covalent or adsorptive bond.

**9.** Composition according to claim 8, wherein the linker compound L comprises at least one carboxyl (-COOH) or carboxylate ($-COO^-$) group as functional group.

**10.** Composition according to claim 8 or 9, wherein the linker compound L comprises at least one guanidino-group ($-NHC(=NH)NH_2$ or $-NHC(=NH_2^+)NH_2$) or amino-group ($-NH_2$ or $-NH_3^+$) group as a functional group.

**11.** Composition according to any of claims 8 to 10, wherein the linker L comprises at least one further functional group L', which is selected from the group -SH, -COOH, $-NH_2$, - guanidino-group ($-NHC(=NH))NH_2$), $-PO_3H_2$, $-PO_2CH_3H$, $-SO_3H$, -OH, $-NR_3^+X^-$.

**12.** Composition according to any of the preceding claims, wherein the pharmaceutically acceptable compounds are conjugated to the nanoparticle by adsorptive attachment.

**13.** Composition according to any of the preceding claims for use as immunostimulant.

**14.** Composition according to any of the preceding claims for the use of the prevention and/or treatment of bovine respiratory disease (BRD).

**15.** Compositions according to any of the preceding claims for perioperative immune stimulation in humans.

Figure 1a

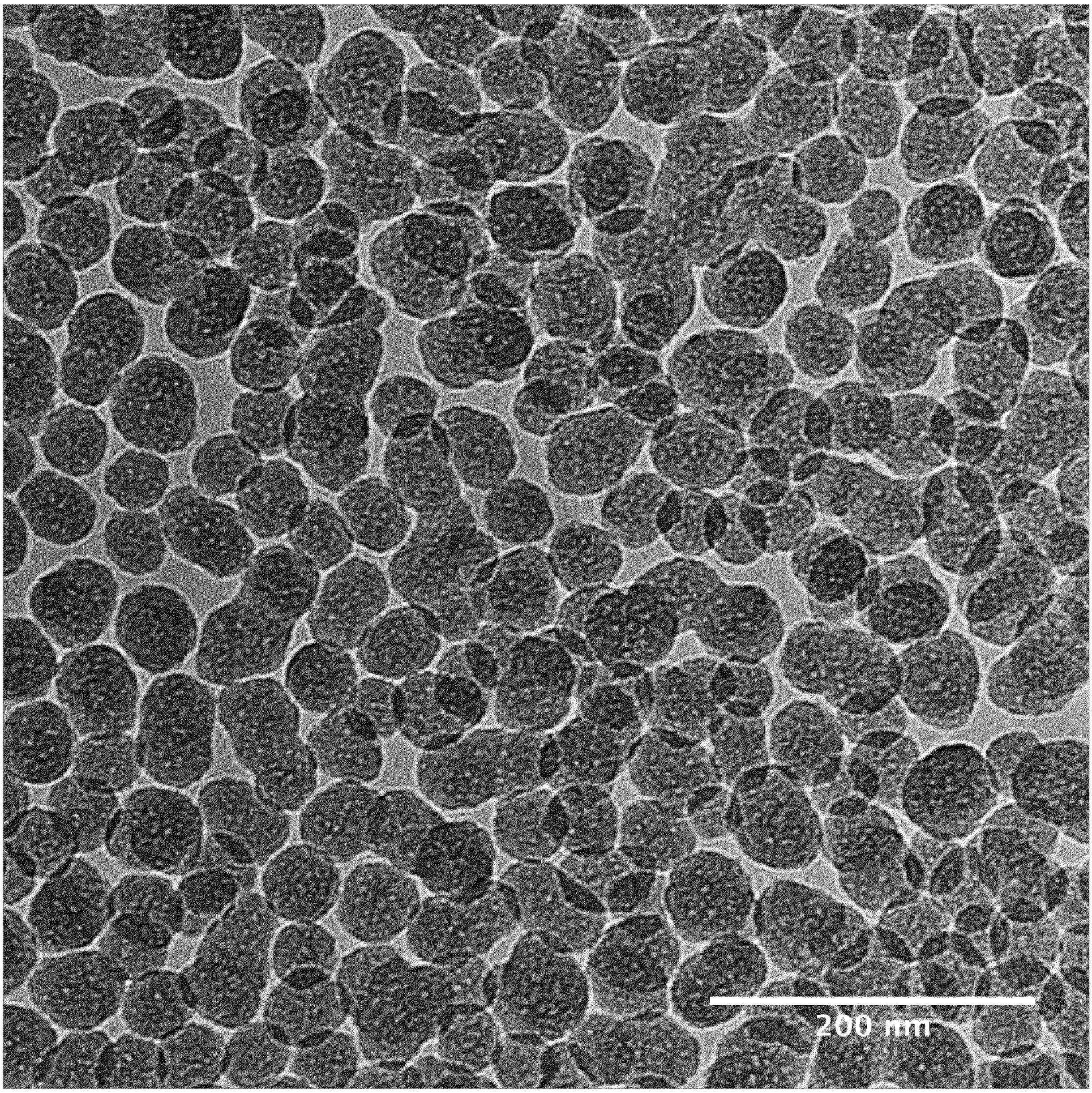

Figure 1b

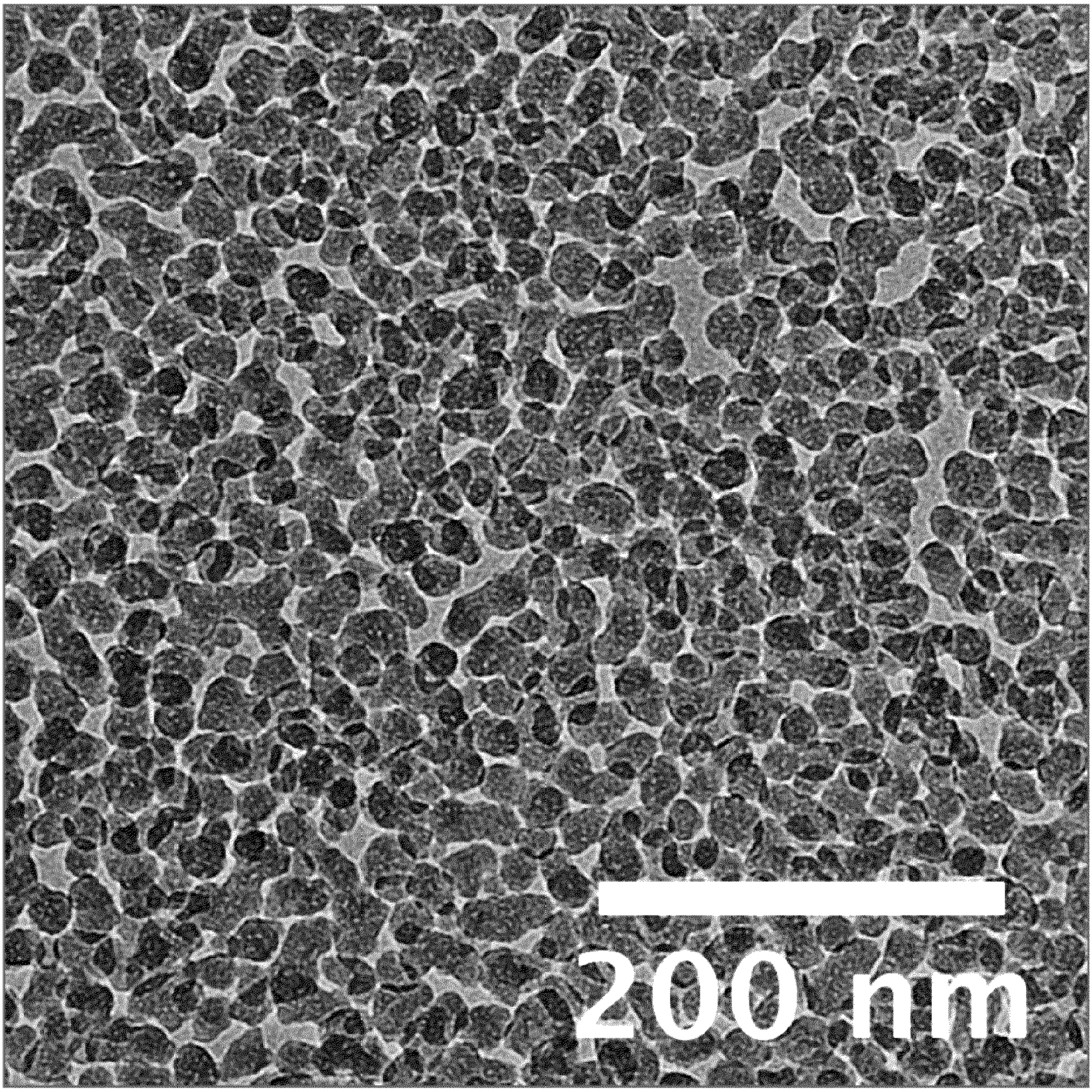

Figure 1c

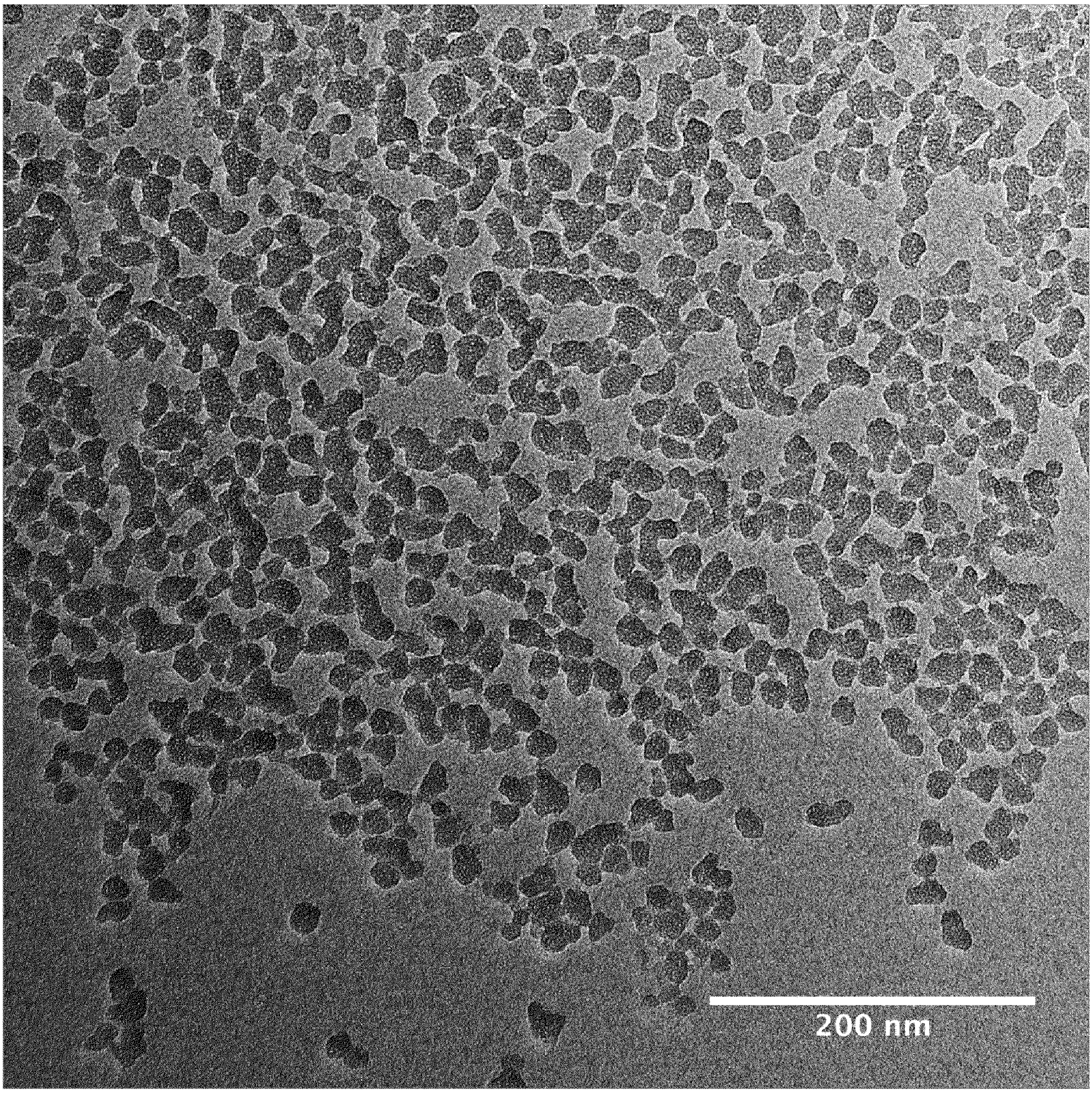

Figure 1d

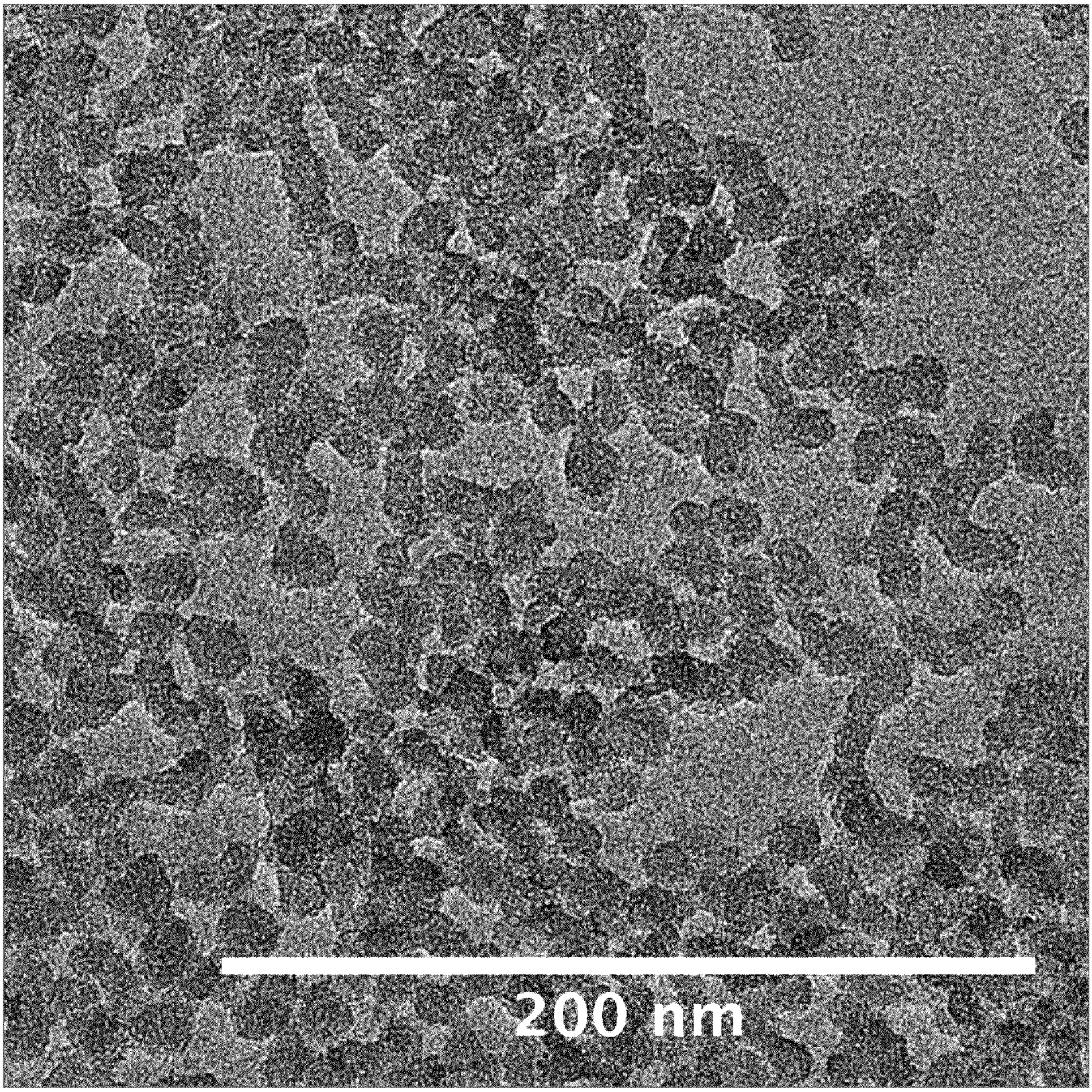

Figure 2

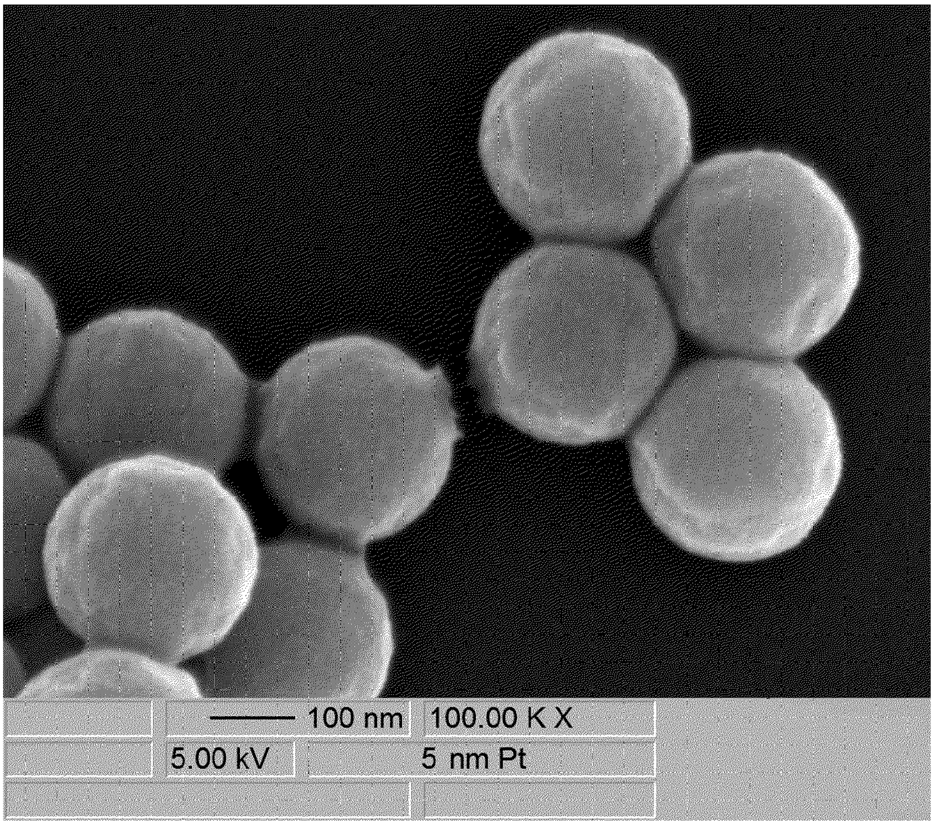


Figure 3

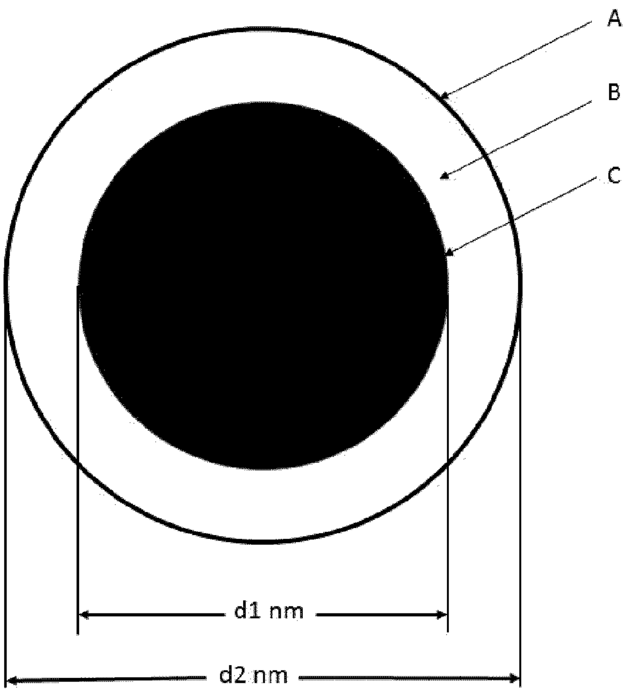

# Figure 4a

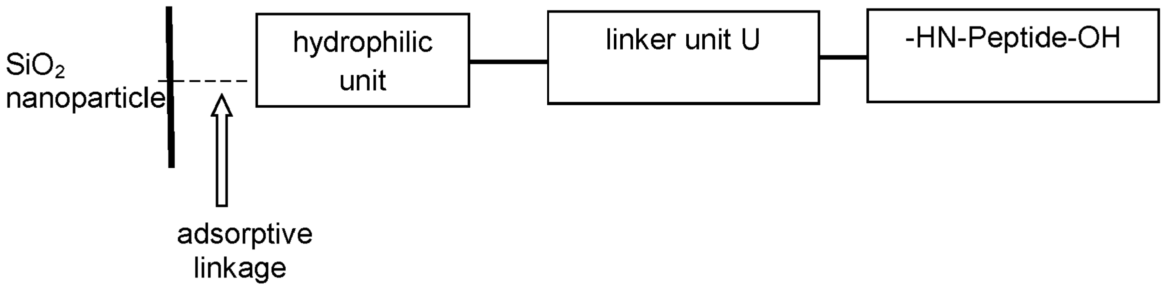


# Figure 4b

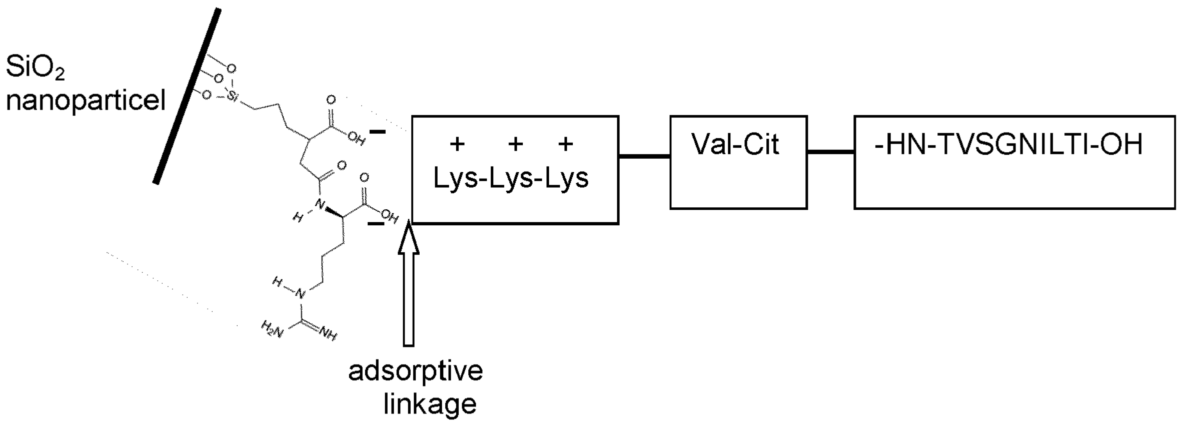

# Figure 5a

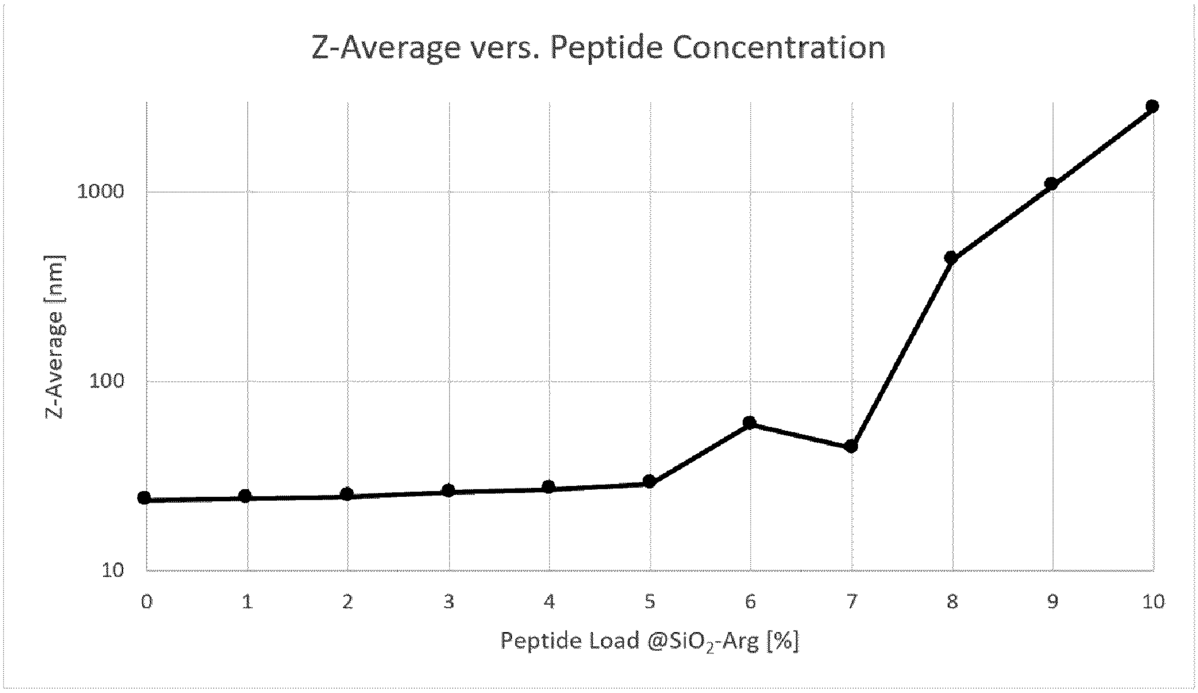


# Figure 5b

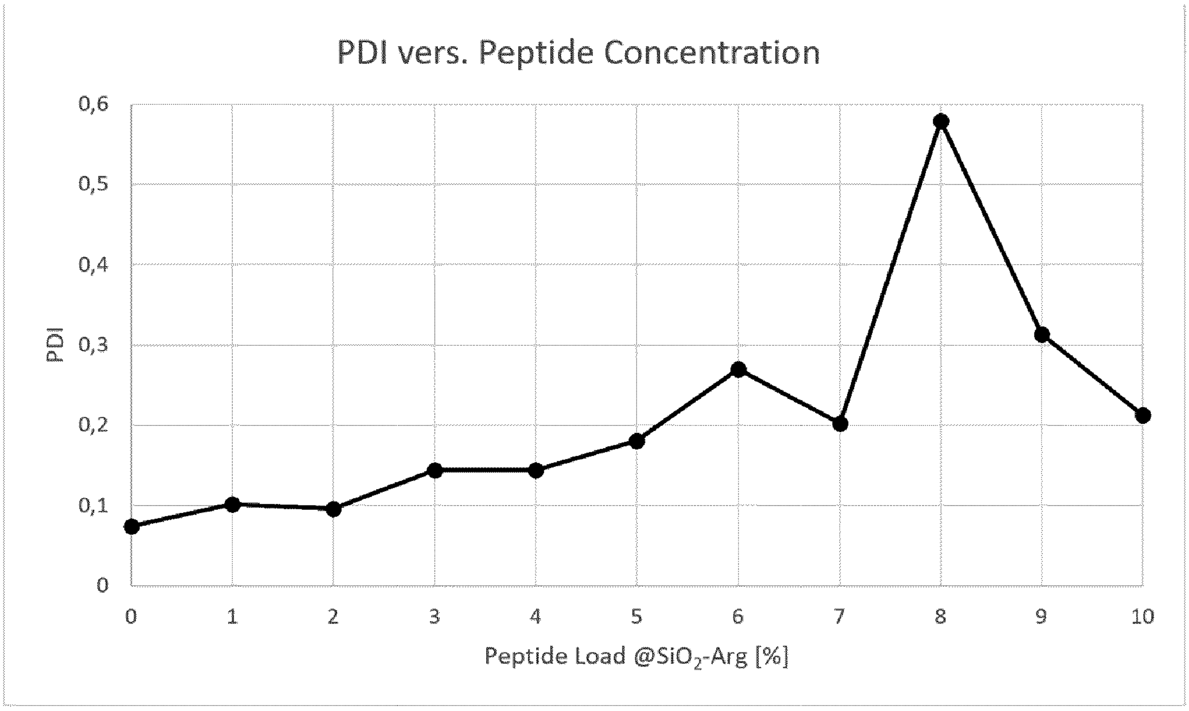

# Figure 6a

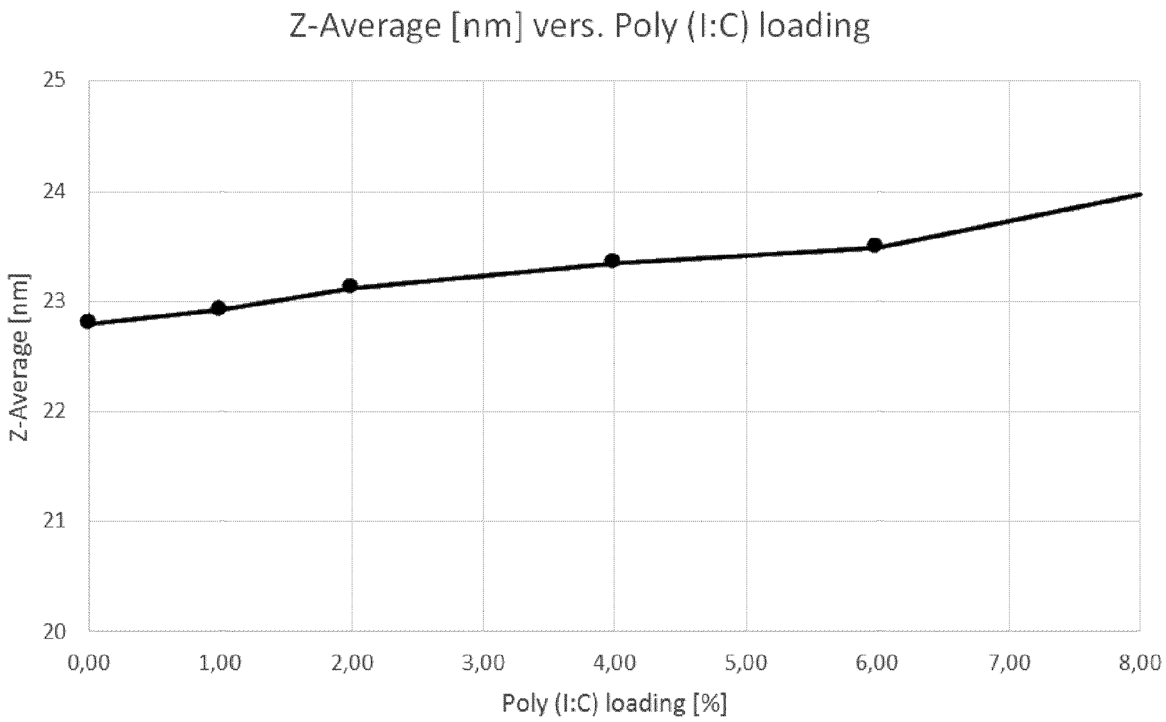


# Figure 6b

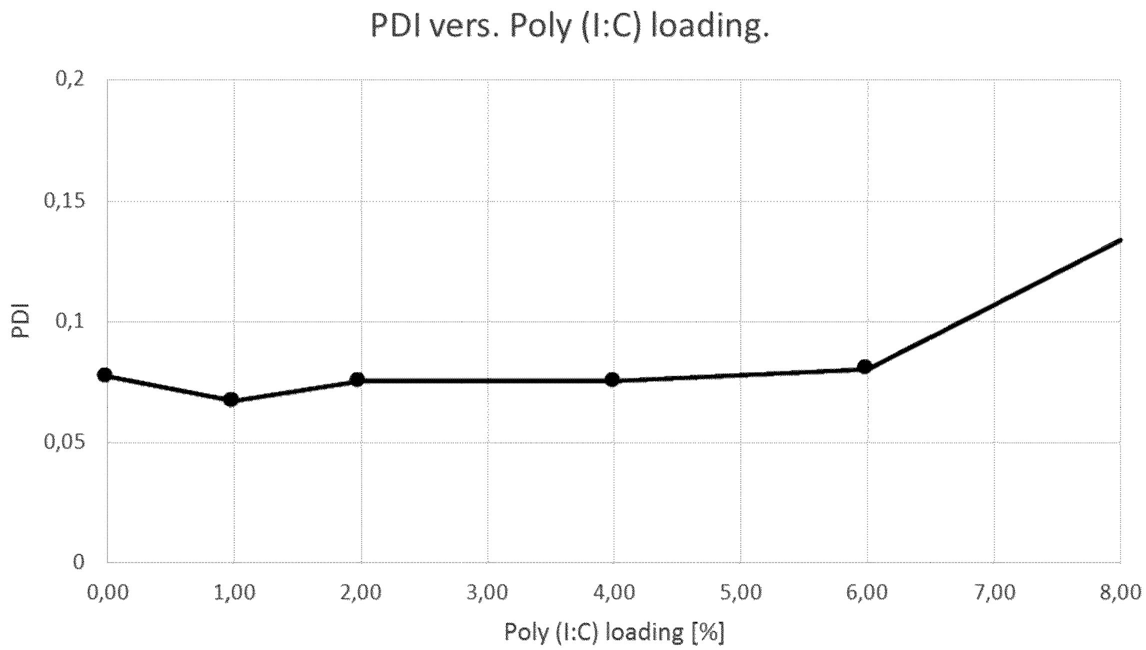

**Figure 7a**

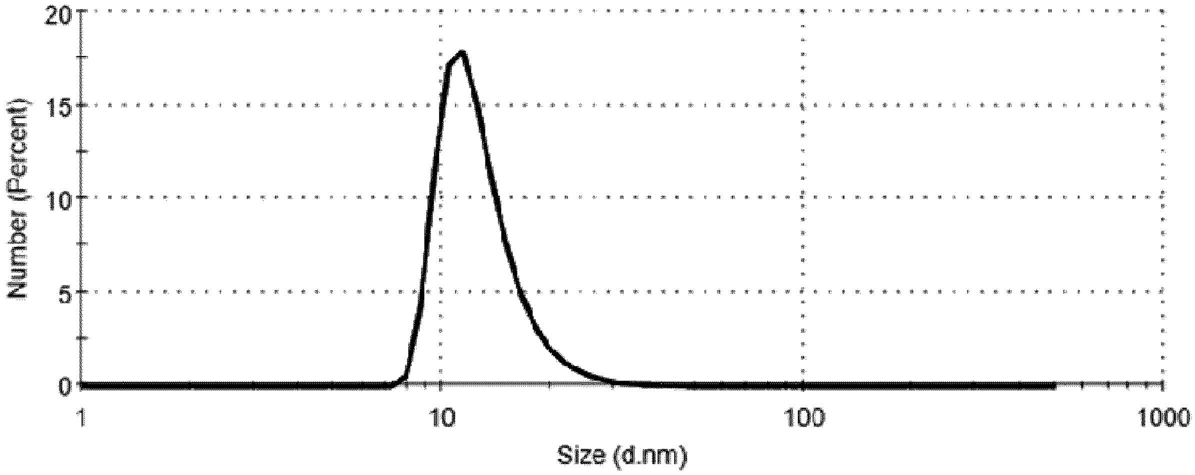


**Figure 7b**

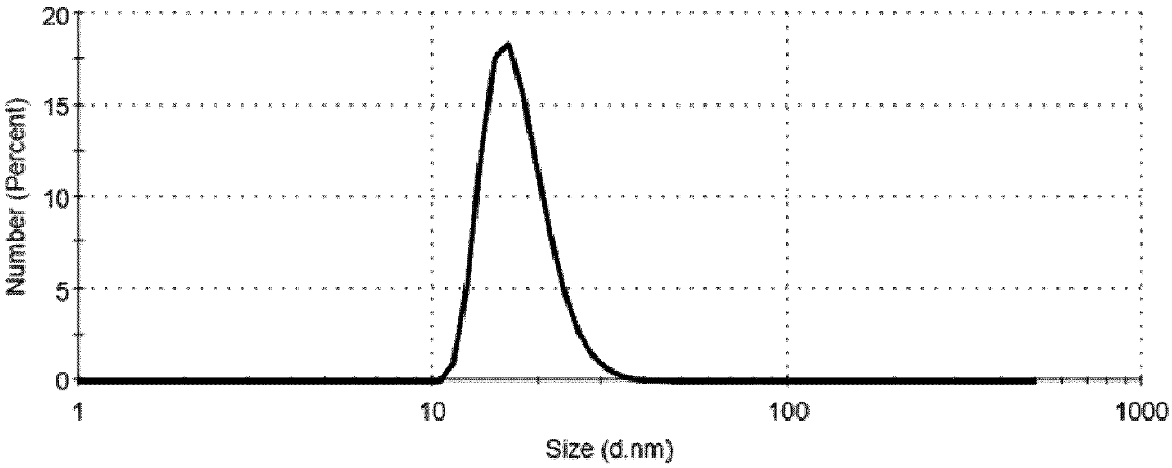

## Figure 7c

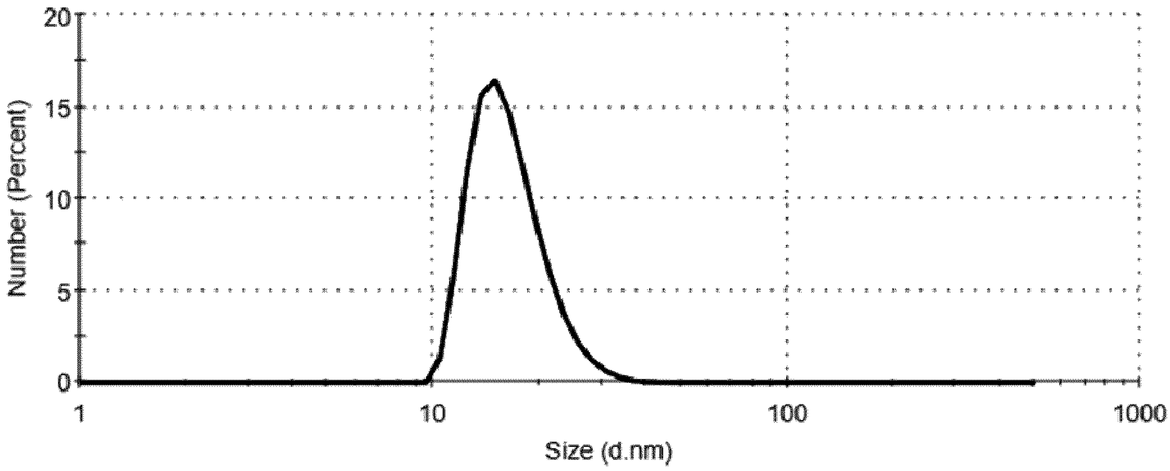


## Figure 7d

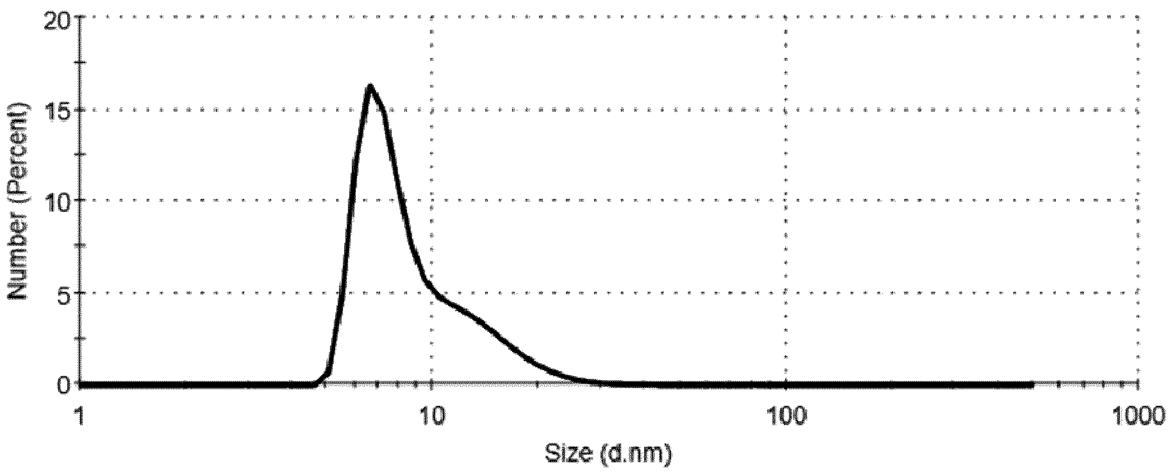

# Figure 8

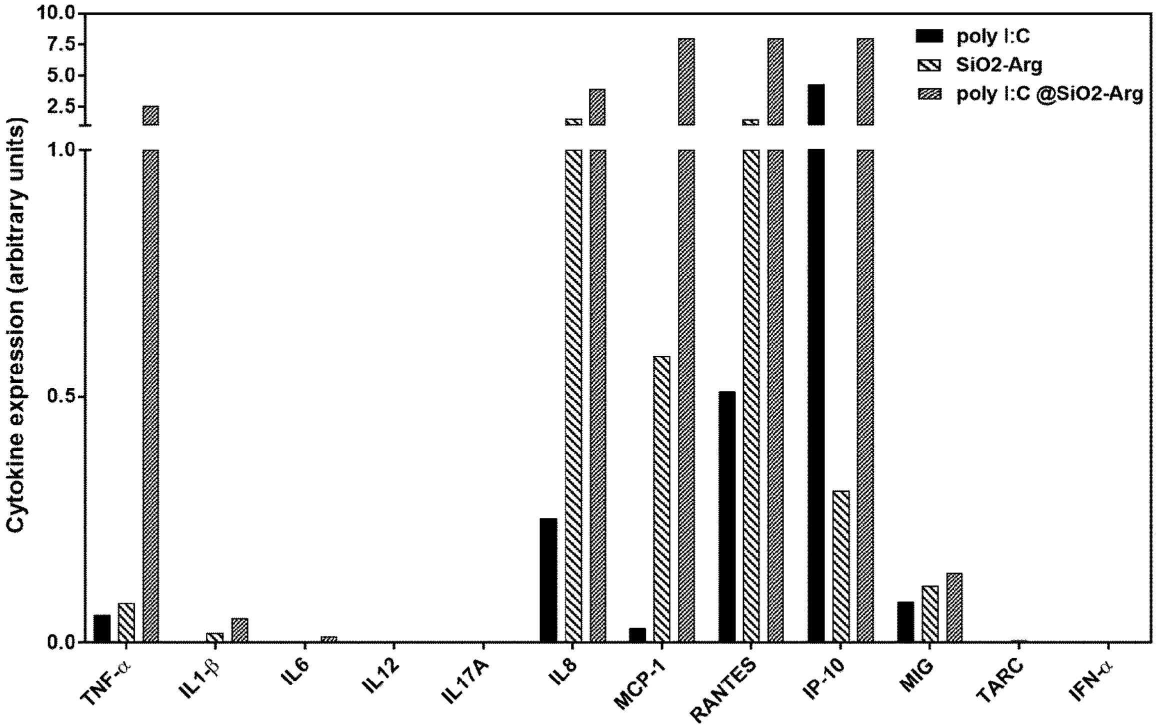


# Figure 9

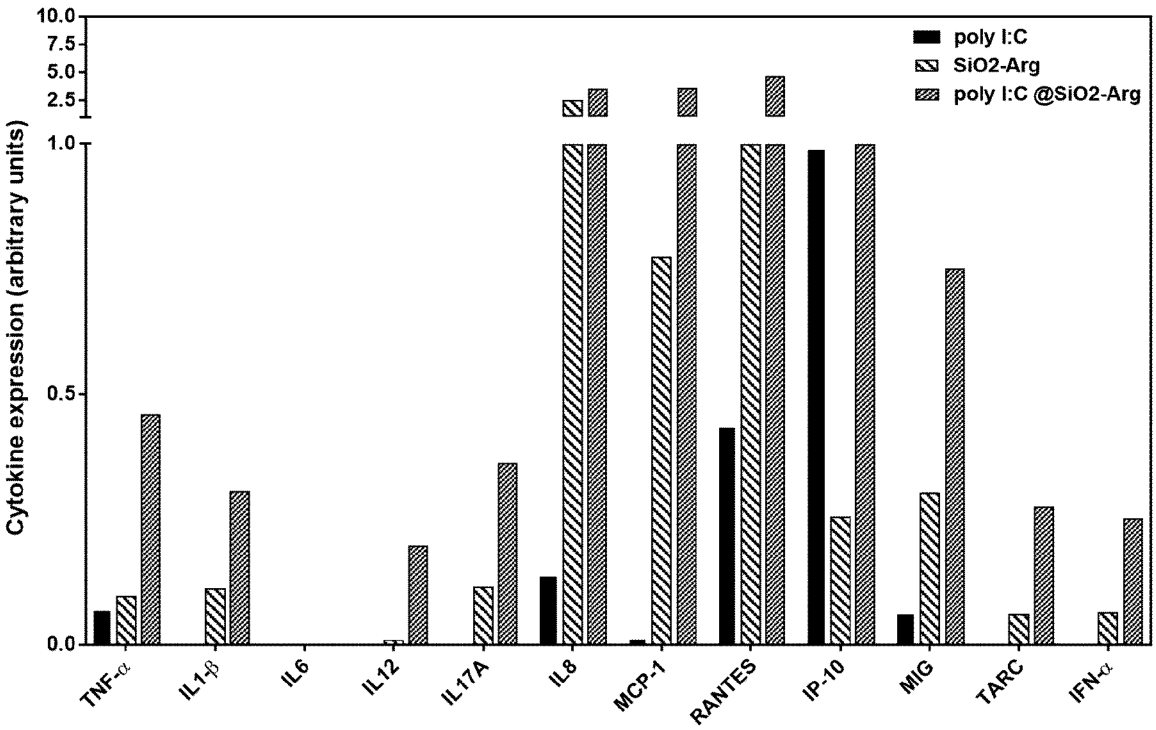

**Figure 10a**

a)

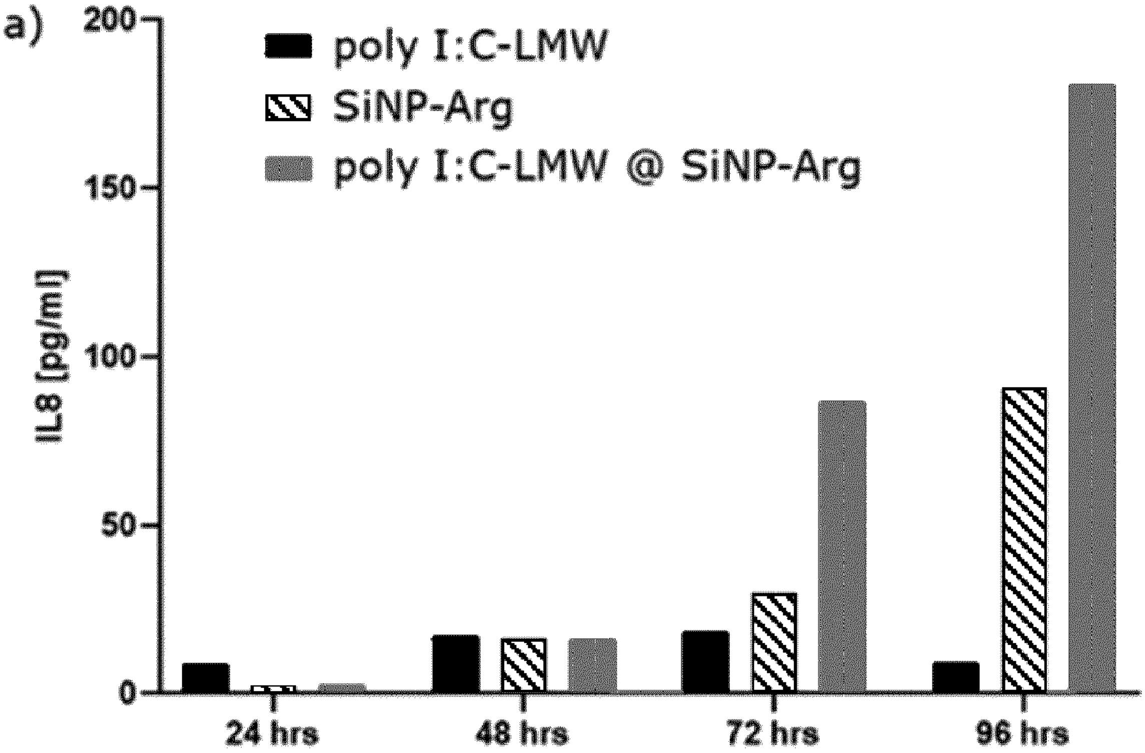


**Figure 10b**

b)

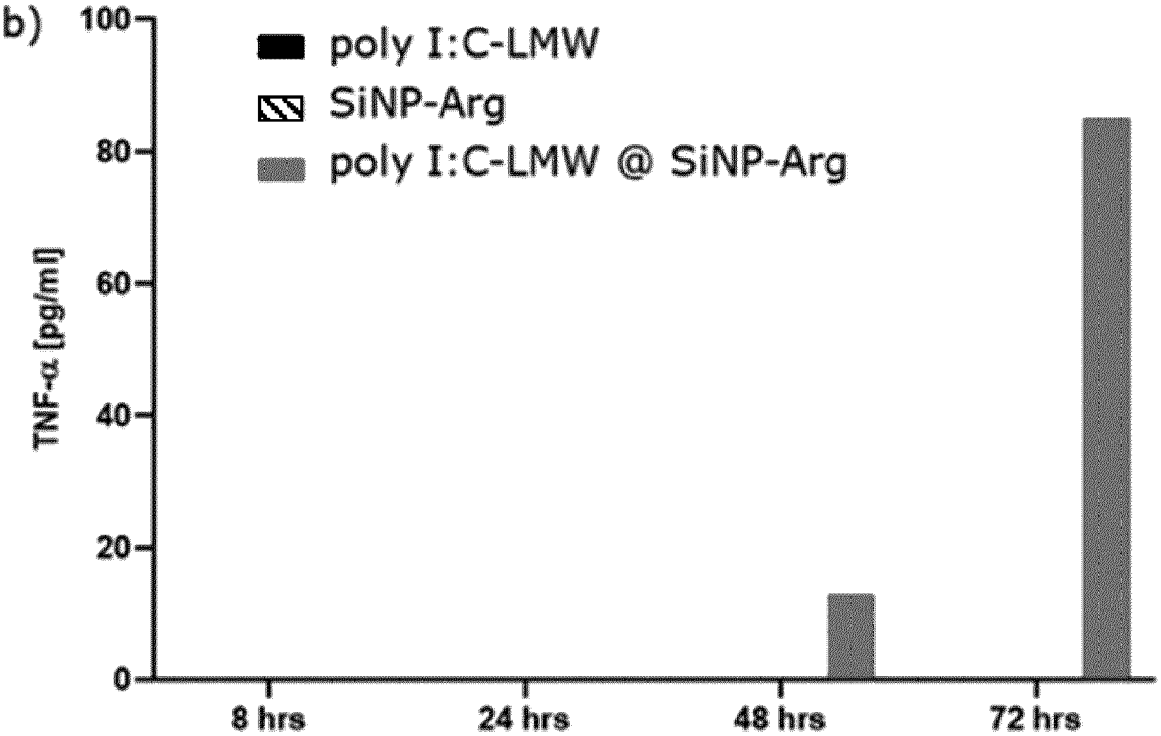

# Figure 11a

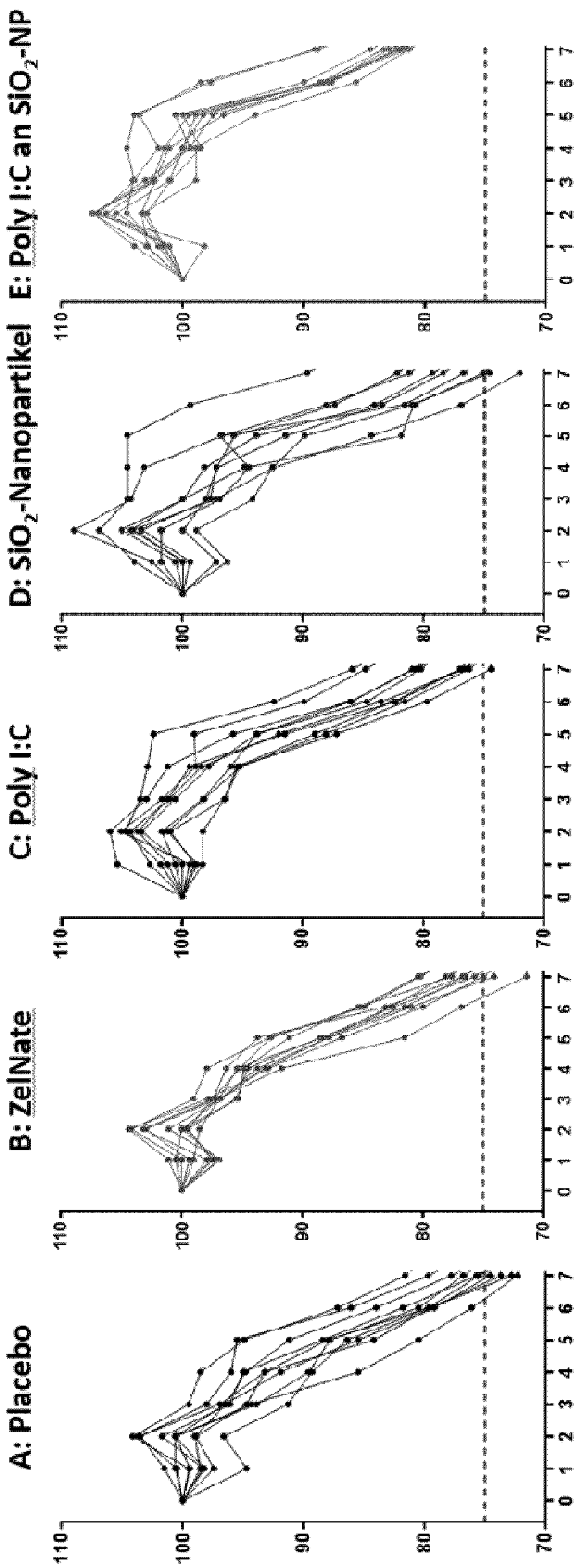
A: Placebo
B: ZelNate
C: Poly I:C
D: SiO$_2$-Nanopartikel
E: Poly I:C an SiO$_2$-NP

**Figure 11b**

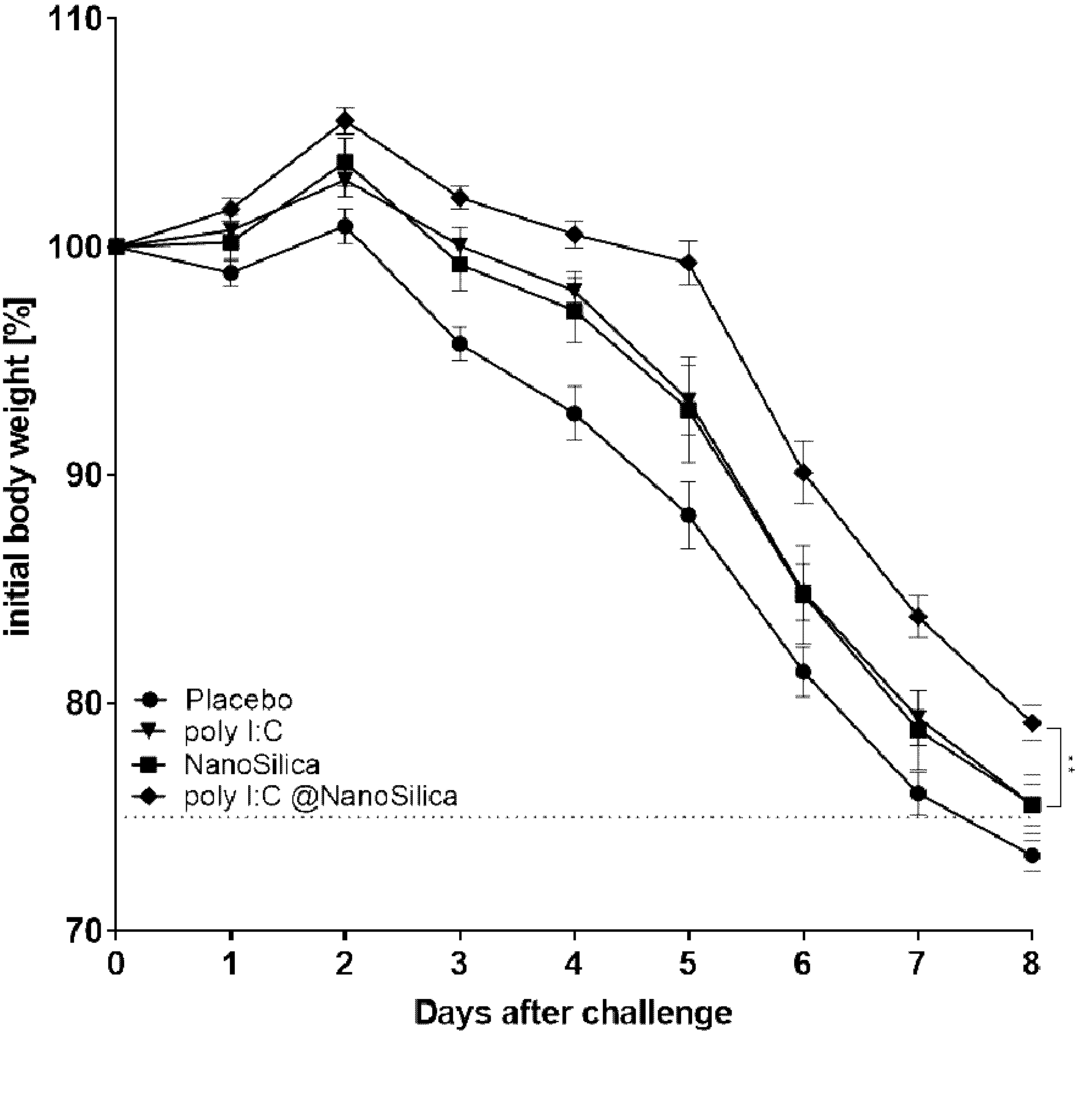
110
100
90
80
70
initial body weight [%]
0
1
2
3
4
5
6
7
8
Days after challenge
Placebo
poly I:C
NanoSilica
poly I:C @NanoSilica
**

# Figure 12

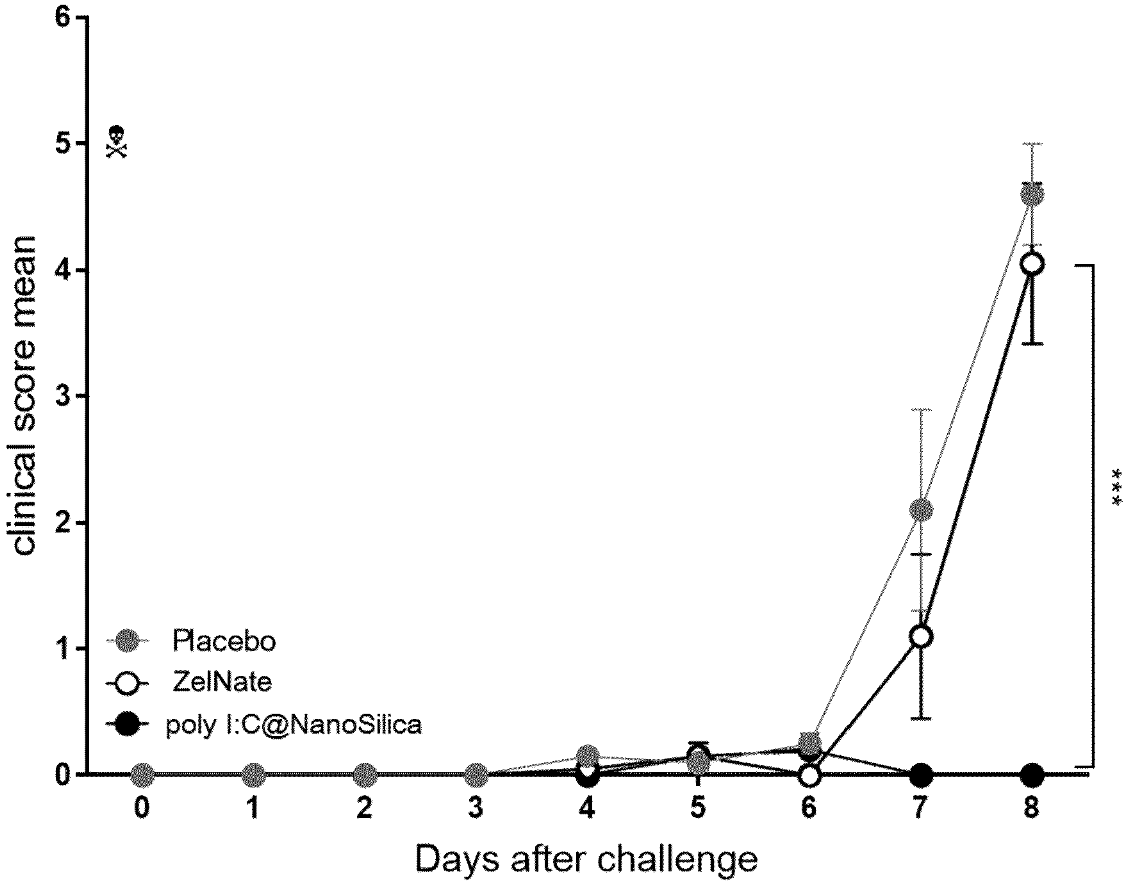
6
5
4
3
2
1
0
clinical score mean
0
1
2
3
4
5
6
7
8
Days after challenge
Placebo
ZelNate
poly I:C@NanoSilica
***

# Figure 13

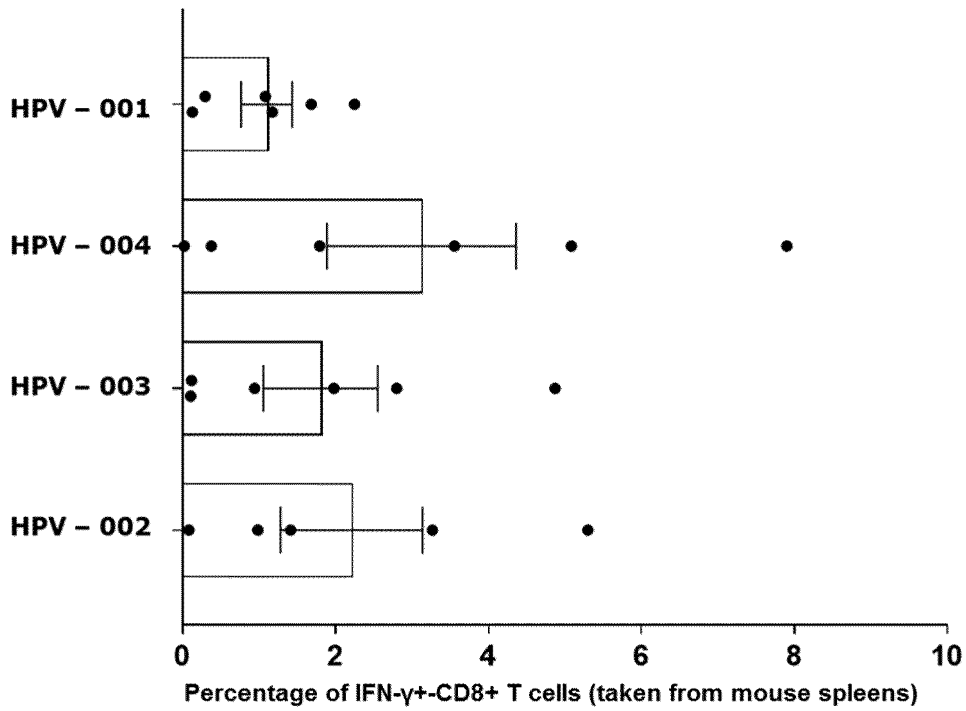
HPV – 001
HPV – 004
HPV – 003
HPV – 002
0
2
4
6
8
10
Percentage of IFN-γ+-CD8+ T cells (taken from mouse spleens)

Figure 14

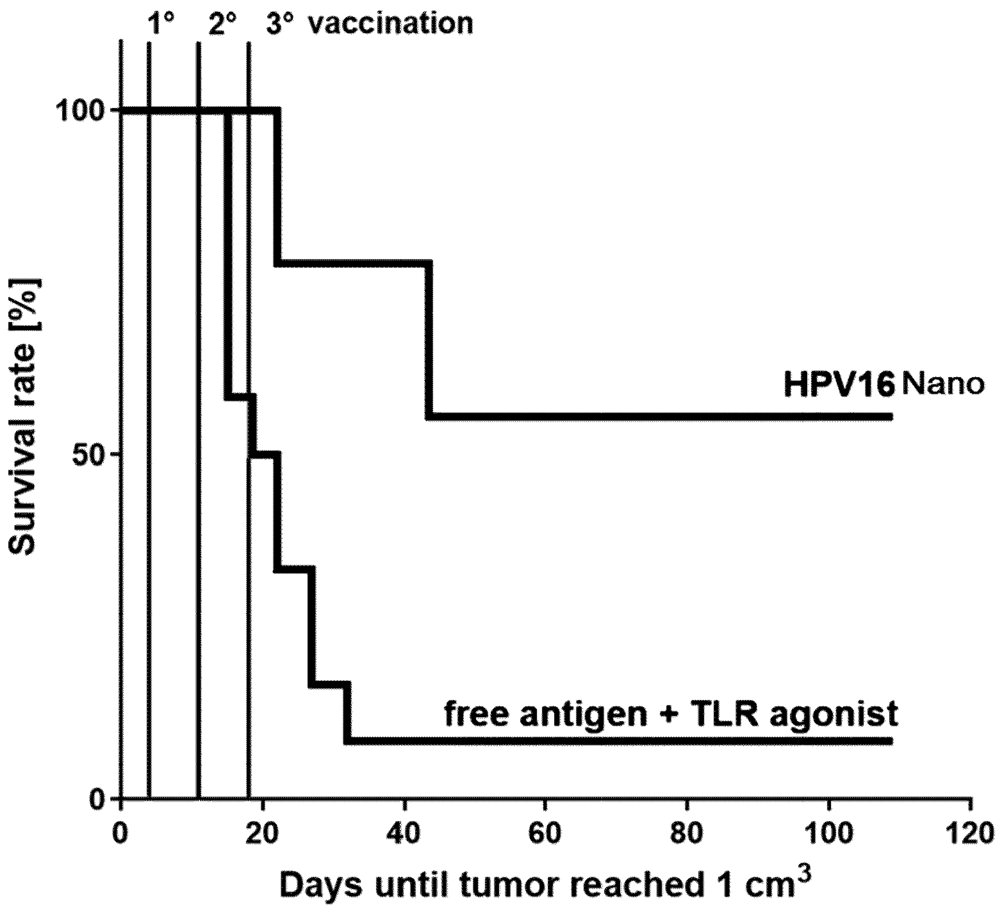
1°
2°
3° vaccination
100
50
0
Survival rate [%]
HPV16 Nano
free antigen + TLR agonist
0
20
40
60
80
100
120
Days until tumor reached 1 cm3

Figure 15a

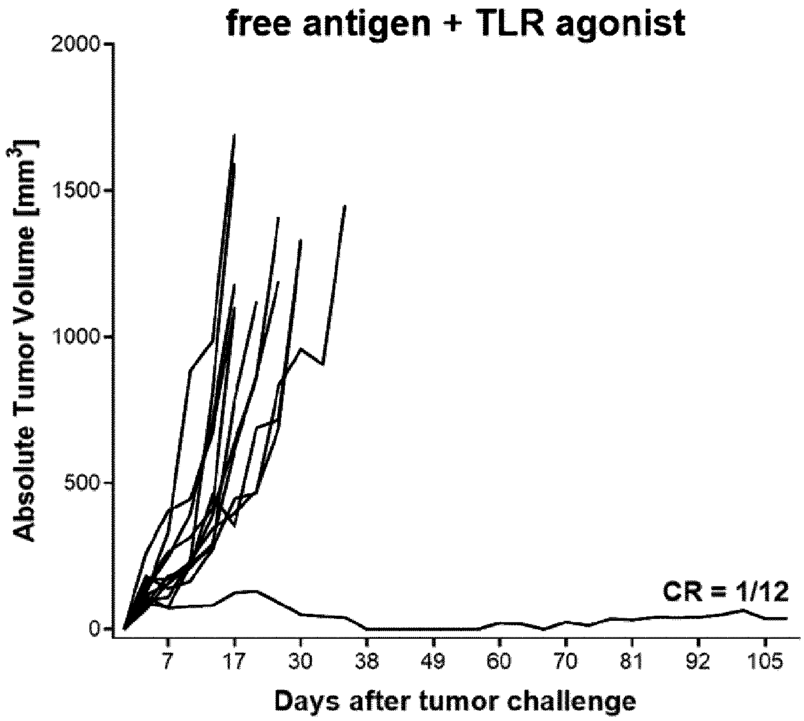


Figure 15b

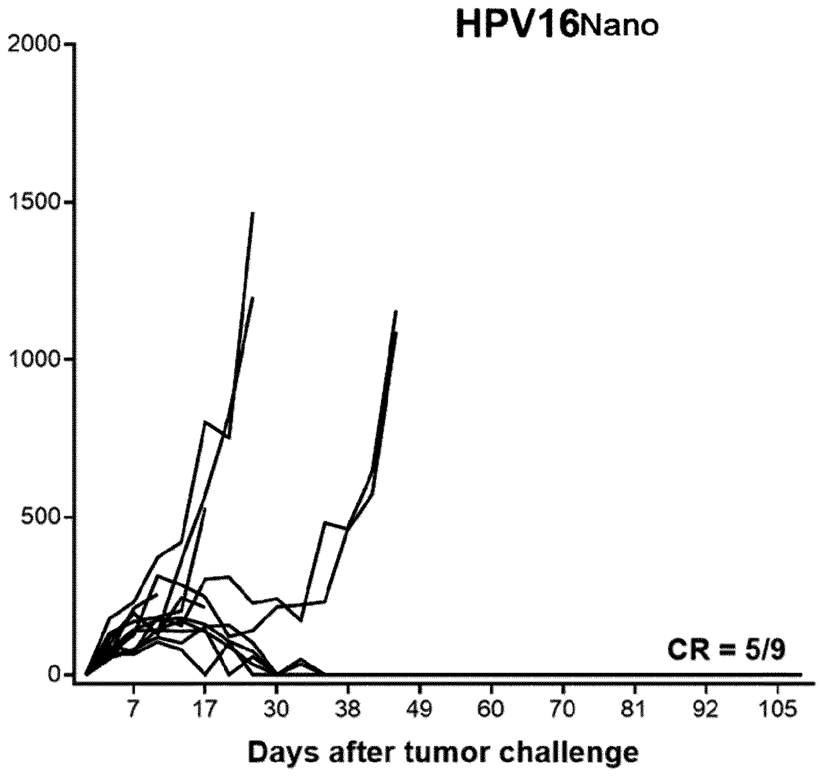

# Figure 16

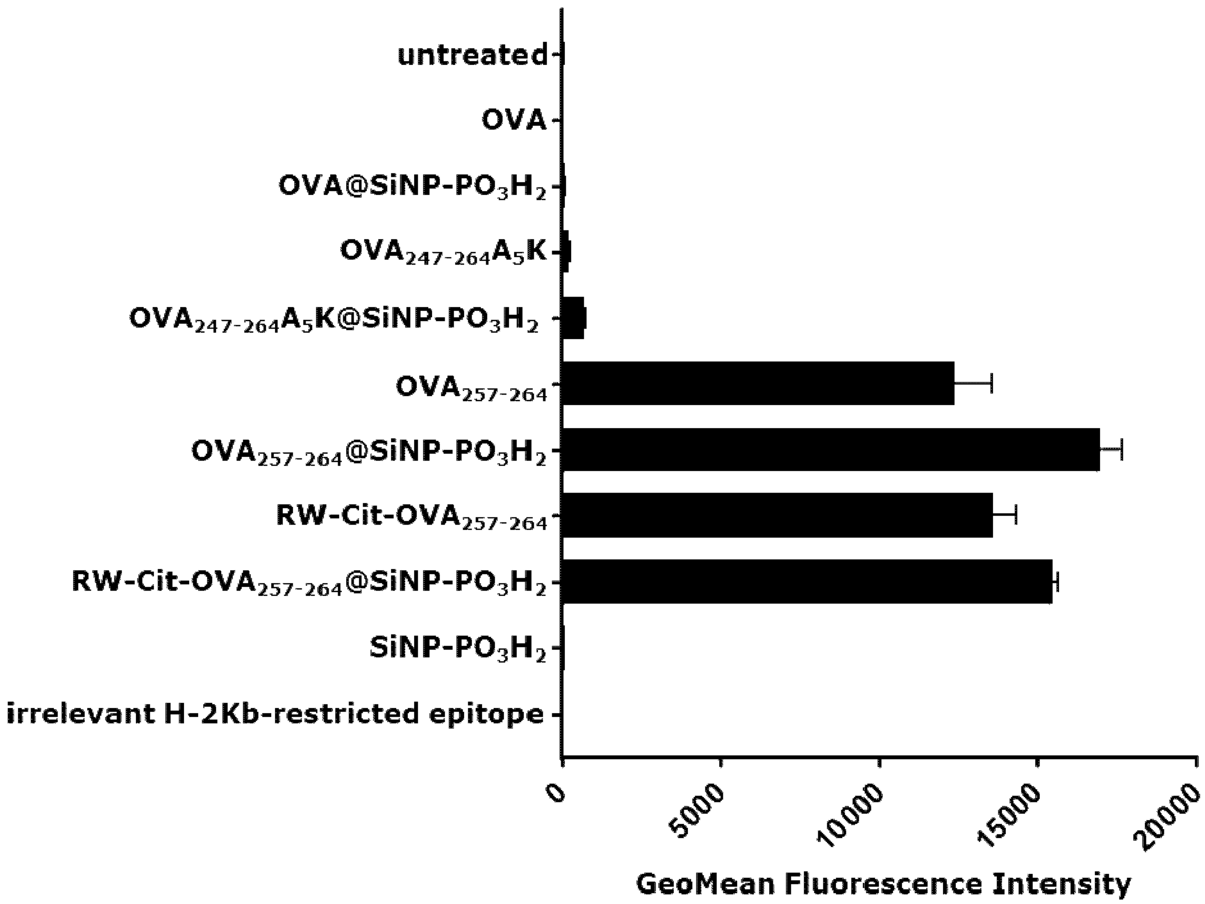
untreated
OVA
OVA@SiNP-PO3H2
OVA247-264A5K
OVA247-264A5K@SiNP-PO3H2
OVA257-264
OVA257-264@SiNP-PO3H2
RW-Cit-OVA257-264
RW-Cit-OVA257-264@SiNP-PO3H2
SiNP-PO3H2
irrelevant H-2Kb-restricted epitope
0
5000
10000
15000
20000
GeoMean Fluorescence Intensity

## Figure 17

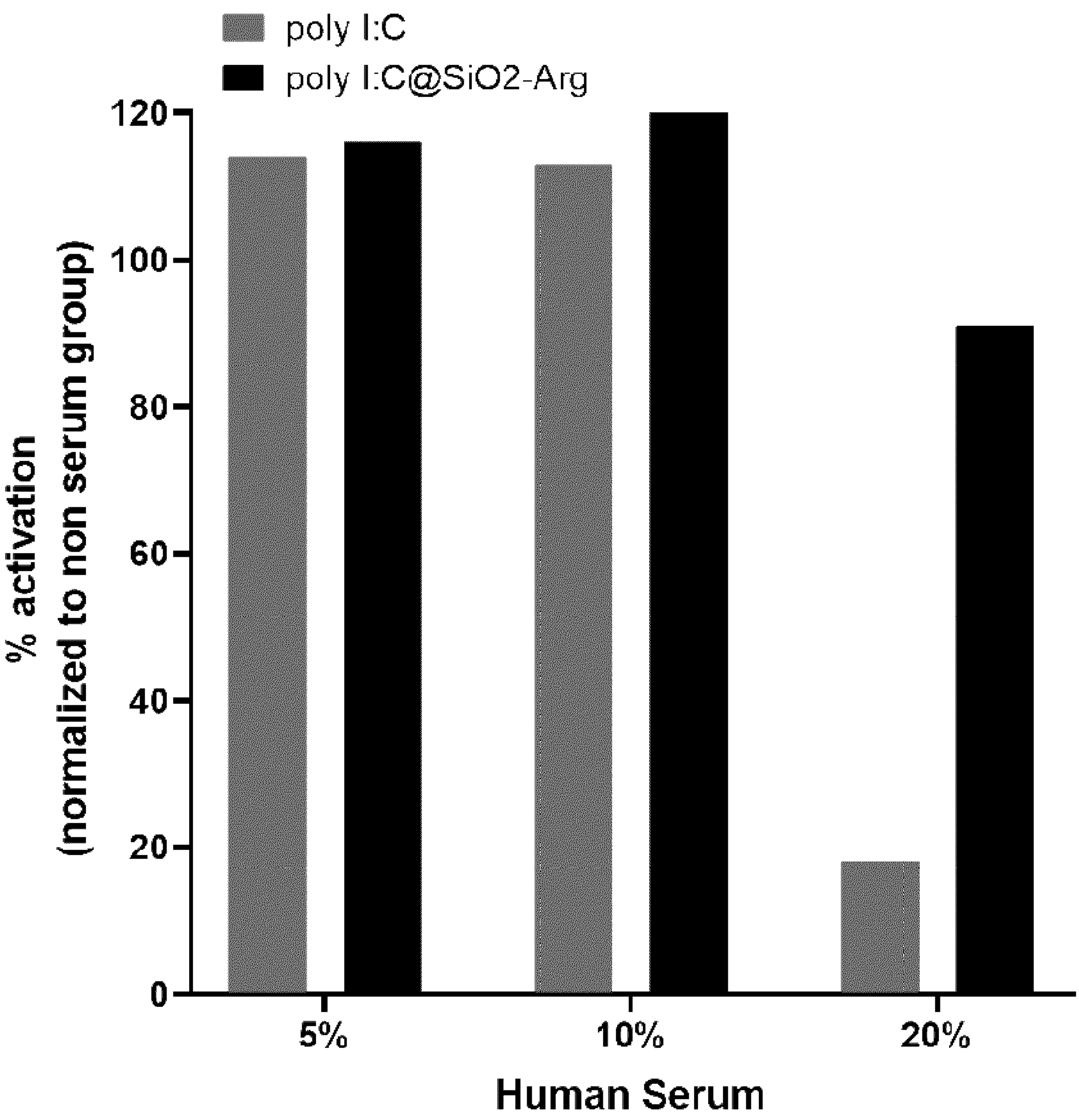
poly I:C
poly I:C@SiO2-Arg
% activation
(normalized to non serum group)
0
20
40
60
80
100
120
5%
10%
20%
Human Serum

Figure 18

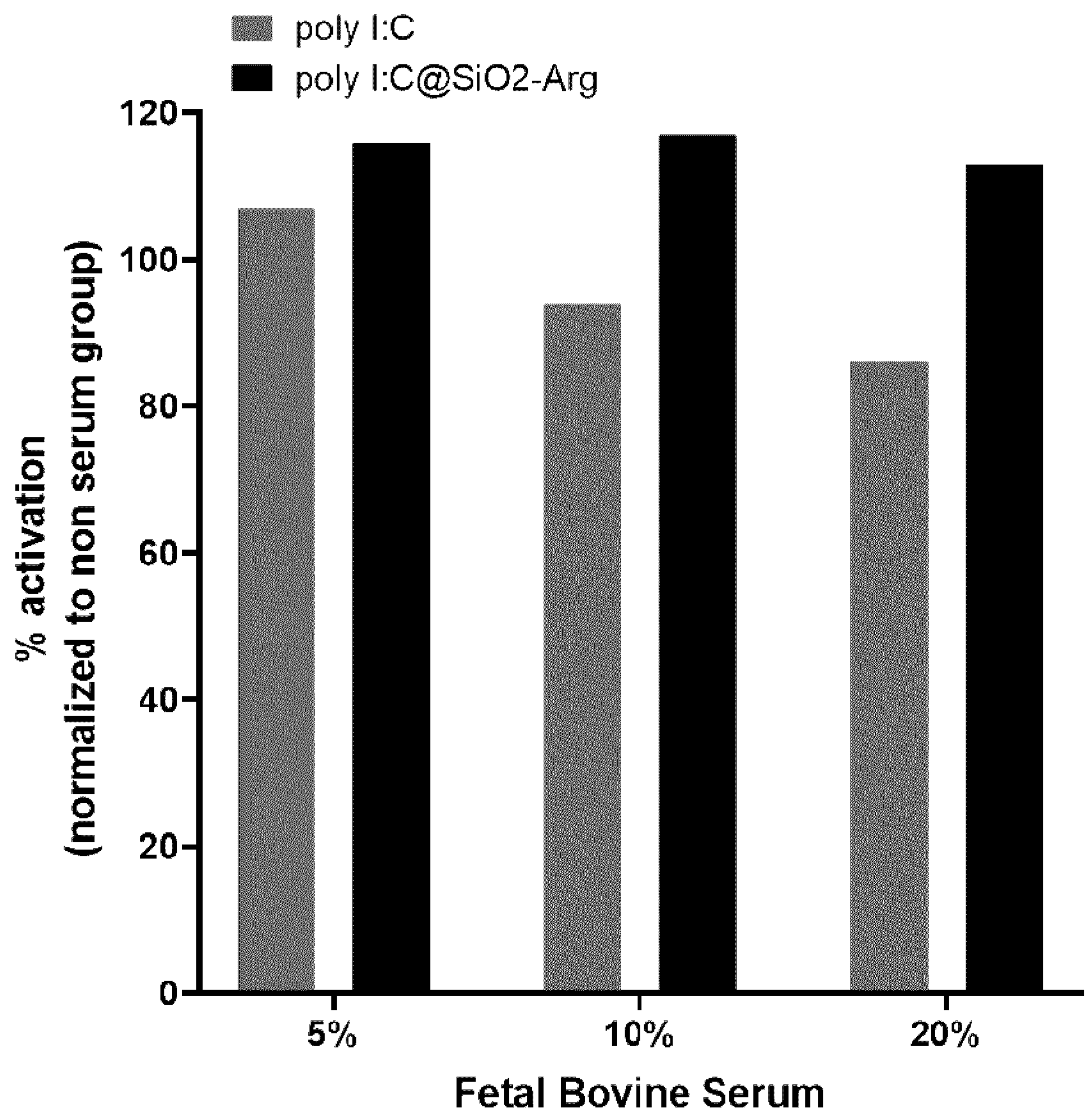
poly I:C
poly I:C@SiO2-Arg
% activation
(normalized to non serum group)
120
100
80
60
40
20
0
5%
10%
20%
Fetal Bovine Serum

Europäisches Patentamt
European Patent Office
Office européen des brevets

# EUROPEAN SEARCH REPORT

Application Number
EP 20 19 6024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2016/090603 A1 (CARNES ERIC CHRISTOPHER [US] ET AL) 31 March 2016 (2016-03-31)<br>* figure 1B *<br>* example 4; tables 2,3 *<br>----- | 1-15 | INV.<br>A61K47/69<br>A61P35/00<br>A61P37/02 |
| Y | SEONG SOO A. AN ET AL: "Analysis of SiO2 nanoparticles binding proteins in rat blood and brain homogenate",<br>INTERNATIONAL JOURNAL OF NANOMEDICINE,<br>vol. 9, no. Suppl.2,<br>15 December 2014 (2014-12-15), pages 207-215, XP055749347,<br>ISSN: 1176-9114, DOI: 10.2147/IJN.S58203<br>* abstract *<br>* page 208, right-hand column *<br>* tables 1-3 *<br>* figures 1-2 *<br>* page 214, left-hand column, paragraph Conclusion *<br>----- | 1-15 | |
| Y | DE 10 2011 018499 A1 (EMC MICROCOLLECTIONS GMBH [DE]) 25 October 2012 (2012-10-25)<br>* examples *<br>* figures *<br>----- | 1-15 | TECHNICAL FIELDS SEARCHED (IPC)<br>A61K<br>A61P |
| Y | WO 2019/097226 A1 (N4 PHARMA UK LTD [GB]) 23 May 2019 (2019-05-23)<br>* examples 2,3 *<br>----- | 1-15 | |
| Y | ES 2 366 841 A1 (CONSEJO SUPERIOR INVESTIGACION [ES]; UNIV VALENCIA POLITECNICA ET AL.)<br>26 October 2011 (2011-10-26)<br>* figure 1; examples *<br>-----<br>-/-- | 1-15 | |

The present search report has been drawn up for all claims

1

| Place of search | Date of completion of the search | Examiner |
|---|---|---|
| The Hague | 19 February 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 19 6024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2006/017476 A2 (UNIV NEW YORK STATE RES FOUND [US]; PRASAD PARAS N [US] ET AL.) 16 February 2006 (2006-02-16)<br>* examples *<br>* figures * | 1-15 | |
| Y | SHAKIBA SHAHABI ET AL: "Utilizing the protein corona around silica nanoparticles for dual drug loading and release", NANOSCALE, vol. 7, no. 39, 2015, pages 16251-16265, XP055749759, ISSN: 2040-3364, DOI: 10.1039/C5NR04726A<br>* abstract *<br>* figures; table 1 *<br>* page 16264, left-hand column, paragraph Conclusions * | 1-15 | |
| Y | WO 00/56288 A1 (INST NEUE MAT GEMEIN GMBH [DE] ET AL.)<br>28 September 2000 (2000-09-28)<br>* examples *<br>* claims * | 1-15 | TECHNICAL FIELDS SEARCHED (IPC) |
| Y | WO 2017/223085 A2 (UNIV MICHIGAN REGENTS [US]) 28 December 2017 (2017-12-28)<br>* example VII * | 1-15 | |

-/--

The present search report has been drawn up for all claims

1

| Place of search | Date of completion of the search | Examiner |
|---|---|---|
| The Hague | 19 February 2021 | Dullaart, Anwyn |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 20 19 6024

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | ULTIMO AMELIA ET AL: "Targeting Innate Immunity with dsRNA-Conjugated Mesoporous Silica Nanoparticles Promotes Antitumor Effects on Breast Cancer Cells", CHEMISTRY A EUROPEAN JOURNAL, vol. 22, no. 5, 7 January 2016 (2016-01-07), pages 1582-1586, XP055777811, ISSN: 0947-6539, DOI: 10.1002/chem.201504629 Retrieved from the Internet: URL:https://api.wiley.com/onlinelibrary/tdm/v1/articles/10.1002%2Fchem.201504629> * abstract * * page 1583 - page 1585 * * figures * ----- | 1-15 | |
| Y | WANG XIUPENG ET AL: "Synergistic effects of stellated fibrous mesoporous silica and synthetic dsRNA analogues for cancer immunotherapy", CHEMICAL COMMUNICATIONS, vol. 54, no. 9, 2018, pages 1057-1060, XP055777800, ISSN: 1359-7345, DOI: 10.1039/C7CC08222C Retrieved from the Internet: URL:https://pubs.rsc.org/en/content/articlepdf/2018/cc/c7cc08222c> * abstract * * figures * * page 1059 * ----- | 1-15 | TECHNICAL FIELDS SEARCHED (IPC) |
| Y | WO 2020/037433 A1 (MIREXUS BIOTECHNOLOGIES INC [CA]) 27 February 2020 (2020-02-27) * examples * ----- | 1-15 | |

1

The present search report has been drawn up for all claims

| Place of search | Date of completion of the search | Examiner |
|---|---|---|
| The Hague | 19 February 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 20 19 6024

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2021

| Patent document cited in search report | Publication date | Patent family member(s) | Publication date |
|---|---|---|---|
| US 2016090603 A1 | 31-03-2016 | US 2016090603 A1 | 31-03-2016 |
| | | US 2018028686 A1 | 01-02-2018 |
| | | US 2018049984 A1 | 22-02-2018 |
| | | WO 2016054225 A1 | 07-04-2016 |
| DE 102011018499 A1 | 25-10-2012 | DE 102011018499 A1 | 25-10-2012 |
| | | EP 2701740 A1 | 05-03-2014 |
| | | WO 2012146364 A1 | 01-11-2012 |
| WO 2019097226 A1 | 23-05-2019 | AU 2018366384 A1 | 04-06-2020 |
| | | CA 3082682 A1 | 23-05-2019 |
| | | CN 111601771 A | 28-08-2020 |
| | | EP 3710404 A1 | 23-09-2020 |
| | | US 2020392005 A1 | 17-12-2020 |
| | | WO 2019097226 A1 | 23-05-2019 |
| ES 2366841 A1 | 26-10-2011 | ES 2366841 A1 | 26-10-2011 |
| | | WO 2011124739 A1 | 13-10-2011 |
| WO 2006017476 A2 | 16-02-2006 | US 2006088599 A1 | 27-04-2006 |
| | | WO 2006017476 A2 | 16-02-2006 |
| WO 0056288 A1 | 28-09-2000 | AT 297716 T | 15-07-2005 |
| | | DE 19912502 A1 | 21-09-2000 |
| | | EP 1162955 A1 | 19-12-2001 |
| | | WO 0056288 A1 | 28-09-2000 |
| WO 2017223085 A2 | 28-12-2017 | CA 3028721 A1 | 28-12-2017 |
| | | EP 3471778 A2 | 24-04-2019 |
| | | WO 2017223085 A2 | 28-12-2017 |
| WO 2020037433 A1 | 27-02-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006037979 A2
- WO 2013034741 A1
- WO 2013034726 A1
- WO 2011154711 A1
- WO 2010006753 A2
- EP 0216278 B1
- WO 2005085135 A1
- EP 0648503 A
- WO 2007031734 A1
- WO 2015162291 A1
- US 6180095 B1
- US 6214345 B1


### Non-patent literature cited in the description


- *Sci. Rep.,* 12 December 2017, vol. 7 (1), 17479
- *J. Colloid Interface Sci.,* 1968, vol. 26, 62
- Vaccine Design: The Subunit and Adjuvant Approach. Pharmaceutical Biotechnology. Plenum Press, 1995, vol. 6
- *CHEMICAL ABSTRACTS,* 74-79-3
- *CHEMICAL ABSTRACTS,* 93642-68-3
- *CHEMICAL ABSTRACTS,* 757-44-8
- **PAJOT, A. et al.** A mouse model of human adaptive immune functions: HLA-A2.1-/HLA-DR1-transgenic H-2 class I-/class II-knockout mice. *European Journal of Immunology,* 2004, vol. 34, 3060-3069
- **KRUSE, S.** Therapeutic vaccination against HPV-positive tumors in a MHC-humanized mouse model. *Ruperto Carola University Heidelberg,* 2019
- **KRUSE et al.** Therapeutic vaccination using minimal HPV16 epitopes in a novel MHC-humanized. *Oncoimmunology,* 2019, vol. 8 (1), e1524694

SEQUENCE LISTING

<110> Life Science Inkubator Betriebs GmbH & Co. KG
Deutsches Krebsforschungszentrum Stiftung des Öffentlichen Rechtes

<120> Nanoparticles as carrier-system for adjuvants/antigens

<130> Nanoparticles as immune boost

<150> EP20174156.8
<151> 2020-05-12

<160> 11

<170> BiSSAP 1.3.6

<210> 1
<211> 10
<212> PRT
<213> human papillomavirus type 16

<220>
<223> E6 protein aa 29-38

<400> 1
Thr Ile His Asp Ile Ile Leu Glu Cys Val
1 5 10

<210> 2
<211> 10
<212> PRT
<213> human papillomavirus type 16

<220>
<223> E6 protein aa 52-61

<400> 2
Phe Ala Phe Arg Asp Leu Cys Ile Val Tyr
1 5 10

<210> 3
<211> 9
<212> PRT
<213> human papillomavirus type 16

<220>
<223> E7 protein aa 11-19

<400> 3
Tyr Met Leu Asp Leu Gln Pro Glu Thr
1 5

<210> 4
<211> 12
<212> PRT
<213> human papillomavirus type 16

<220>

<223> E7 protein aa 73-84

<400> 4
His Val Asp Ile Arg Thr Leu Glu Asp Leu Leu Met
1 5 10

<210> 5
<211> 9
<212> PRT
<213> human papillomavirus type 16

<220>
<223> E7 protein aa 79-87

<400> 5
Leu Glu Asp Leu Leu Met Gly Thr Leu
1 5

<210> 6
<211> 9
<212> PRT
<213> human papillomavirus type 16

<220>
<223> E7 protein aa 82-90

<400> 6
Leu Leu Met Gly Thr Leu Gly Ile Val
1 5

<210> 7
<211> 11
<212> PRT
<213> human papillomavirus type 18

<220>
<223> E6 protein aa 125-135

<400> 7
Arg Arg Phe His Asn Ile Ala Gly His Tyr Arg
1 5 10

<210> 8
<211> 12
<212> PRT
<213> human papillomavirus type 18

<220>
<223> E7 protein aa 5-16

<400> 8
Lys Ala Thr Leu Gln Asp Ile Val Leu His Leu Glu
1 5 10

<210> 9
<211> 9
<212> PRT
<213> Homo sapiens

```
<220>
<223> Cancer/testis antigen 1 NY-ESO-1 aa 127-135

<400> 9
Thr Val Ser Gly Asn Ile Leu Thr Ile
1               5

<210> 10
<211> 8
<212> PRT
<213> Gallus gallus


<220>
<223> Ovalbumin aa 258-265

<400> 10
Ser Ile Ile Asn Phe Glu Lys Leu
1               5

<210> 11
<211> 22
<212> DNA
<213> Artificial Sequence


<220>
<223> ODN 2395 human/murine TLR9 ligand

<400> 11
tcgtcgtttt cggcgcgcgc cg                                              22
```